(19) Europäisches Patentamt
European Patent Office
Office européen des brevets

(11) EP 4 733 384 A1

(12) **EUROPEAN PATENT APPLICATION**
published in accordance with Art. 153(4) EPC

(43) Date of publication:
**29.04.2026 Bulletin 2026/18**

(21) Application number: **24845554.5**

(22) Date of filing: **19.07.2024**

(51) International Patent Classification (IPC):
***C12N 1/02*** ^(2006.01) ***C12N 5/07*** ^(2010.01)

(52) Cooperative Patent Classification (CPC):
**C12N 1/02; C12N 5/06**

(86) International application number:
**PCT/JP2024/025934**

(87) International publication number:
**WO 2025/023177 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.07.2023 JP 2023122451**

(71) Applicants:
- **Eiken Kagaku Kabushiki Kaisha**
**Tokyo 101-0062 (JP)**
- **Sanyo Chemical Industries, Ltd.**
**Kyoto-shi, Kyoto 605-0995 (JP)**

(72) Inventors:
- **MURAKAMI Yusuke**
**Shimotsuga-gun, Tochigi 329-0114 (JP)**
- **OTA Koji**
**Kyoto-shi, Kyoto 605-0995 (JP)**
- **TOBINAGA Kyohei**
**Kyoto-shi, Kyoto 605-0995 (JP)**

(74) Representative: **Boult Wade Tennant LLP**
**Salisbury Square House**
**8 Salisbury Square**
**London EC4Y 8AP (GB)**

(54) **METHOD FOR PURIFYING EXTRACELLULAR VESICLES AND/OR MEMBRANE VESICLES IN FECES**

(57) An object of the present invention is to provide a method for purifying extracellular vesicles and/or membrane vesicles in feces from a simple, low-cost, and efficient manner. The method for purifying extracellular vesicles and/or membrane vesicles in feces of the present invention includes a step of filtering a sample of the feces with a filter, and a step of bringing the filtered sample into contact with a capturing agent, in which the capturing agent contains a crosslinked polymer including monomer units having an acidic group and/or monomer units having a neutralizing base of the acidic group, and satisfies the predetermined conditions (i) and/or (ii).

EP 4 733 384 A1

## Description

### Technical Field

The present invention relates to a method for purifying extracellular vesicles and/or membrane vesicles in feces.

### Background Art

Extracellular vesicles are lipid bilayer vesicles secreted by cells, encapsulating proteins, nucleic acids, and the like, transporting these substances, and being involved in various physiological phenomena. The extracellular vesicles are present in most body fluids and are also abundantly present in feces. In addition to the extracellular vesicles, feces contain membrane vesicles released by intestinal bacteria or the like, which have functions similar to those of extracellular vesicles. Since the extracellular vesicles and membrane vesicles have the potential to be applied to diagnoses of diseases and the like, they have been attracting attention in recent years, for example, in the medical field. Therefore, it is desired to develop methods for purifying extracellular vesicles and/or membrane vesicles from biological samples. However, studies on extracellular vesicles and membrane vesicles in feces are limited.

Compared with urine or blood, feces contain many impurities, making it difficult to purify extracellular vesicles and membrane vesicles from feces. Conventionally, a purification method combining high-speed filtration and sucrose density gradient ultracentrifugation has been reported (for example, Non-Patent Literature 1). However, this method requires a long processing time because it uses ultracentrifugation, and further requires expensive and large equipment.

On the other hand, methods for separating exosomes or lipid bilayers from biological samples other than feces are known. For example, Patent Literature 1 discloses a method for separating exosomes from a biological sample, the method including a process of bringing a biological sample into contact with a superabsorbent polymer to obtain a superabsorbent polymer gel containing exosomes, and a process of mixing the superabsorbent polymer gel with a salt to recover the exosomes. It is also described that the superabsorbent polymer preferably has repeating units represented by a predetermined structural formula. In addition, Patent Literature 2 discloses a method for separating a substance having a lipid bilayer from a biological sample, the method including a process of bringing the biological sample into contact with a predetermined crosslinked polymer to obtain a polymer gel containing a substance having a lipid bilayer, and a process of mixing the polymer gel with a salt to recover the substance having a lipid bilayer. As the predetermined crosslinked polymer, it is a crosslinked polymer that includes monomer units having an acidic group and/or a neutralizing base thereof and contains a compound having at least one group selected from the group consisting of a cationic group and a hydroxyl group. However, neither Patent Literature 1 nor Patent Literature 2 describes or suggests that extracellular vesicles and membrane vesicles can be purified from feces.

### Citation List

#### Patent Literature

[Patent Literature 1] International Publication No. WO 2021/251462
[Patent Literature 2] International Publication No. WO 2022/211006

#### Non-Patent Literature

[Non-Patent Literature 1] Yang C, et al., "Isolation and Characterization of Exosomes from Mouse Feces." Bio Protoc. 2020 Apr 20; 10(8): e3584.

### Summary of Invention

#### Technical Problem

An object of the present invention is to provide a method for purifying extracellular vesicles and/or membrane vesicles in feces from a simple, low-cost, and efficient manner.

#### Solution to Problem

The present inventors have conducted extensive studies to solve the above problem and, as a result, have found

that by filtering a supernatant of a fecal suspension with a filter and capturing the filtered sample using a predetermined capturing agent, the capture efficiency of extracellular vesicles is greatly improved, thereby completing the present invention.

That is, the present invention relates to, for example, the following inventions.

[1] A method for purifying extracellular vesicles and/or membrane vesicles in feces, the method including

a step of filtering a sample of the feces with a filter, and a step of bringing the filtered sample into contact with a capturing agent, in which the capturing agent contains a crosslinked polymer including monomer units having an acidic group and/or monomer units having a neutralizing base of the acidic group, and satisfies the following (i) and/or (ii):

(i) the crosslinked polymer includes at least monomer units having a neutralizing base between the acidic group and the first cationic surfactant; and
(ii) the capturing agent further contains a second cationic surfactant (provided that the second cationic surfactant is excluded when the second cationic surfactant is a crosslinked polymer including monomer units having a neutralizing base of the acidic group).

[2] The method according to [1], wherein the monomer units having the acidic group are unsaturated monocarboxylic acids having 3 to 10 carbon atoms, and the monomer units having the neutralizing base of the acidic group are unsaturated monocarboxylate salts having 3 to 10 carbon atoms.
[3] The method according to [1] or [2], wherein the monomer units having the acidic group are acrylic acid, and the monomer units having the neutralizing base of the acidic group are acrylates.
[4] The method according to any one of [1] to [3], wherein a cationic group in the cationic surfactant is $-N^+R_3$ (wherein each R independently represents a hydrogen atom or a hydrocarbon group having 1 to 6 carbon atoms, the hydrocarbon group being optionally substituted).
[5] The method according to any one of [1] to [4], wherein the cationic surfactant has an aliphatic hydrocarbon group having 12 to 30 carbon atoms, the aliphatic hydrocarbon group being optionally substituted.
[6] The method according to any one of [1] to [5], wherein the cationic surfactant is hexadecyltrimethylammonium chloride.
[7] The method according to any one of [1] to [6], wherein a pore size of the filter is 0.8 μm or less.

**Advantageous Effects of Invention**

According to the present invention, it is possible to provide a method for purifying extracellular vesicles and/or membrane vesicles in feces from a simple, low-cost, and efficient manner.

**Brief Description of Drawings**

FIG. 1 is a graph illustrating CD9/CD63 luminescence values in a supernatant before reaction and in the supernatant after reaction (flow-through), when the supernatant of urine is reacted with a capturing agent (A), or when the supernatant of a fecal suspension is reacted with a capturing agent (B).
FIG. 2 is a graph illustrating CD9/CD63 luminescence values in the supernatant of a fecal suspension before reaction and in the supernatant after reaction (flow-through), when the supernatant of the fecal suspension is reacted with a capturing agent without filtering the supernatant of the fecal suspension with a filter (A), or when the supernatant of the fecal suspension is filtered with a filter and reacted with a capturing agent (B).
FIG. 3 is a graph illustrating CD9/CD63 luminescence values in the supernatant before treatment and in the supernatant after treatment (flow-through), when the supernatant of serum is treated with a predetermined kit (A), or when the supernatant of a fecal suspension is treated with the same kit (B).
FIG. 4 is a graph showing CD9/CD63 luminescence values in the filtrate of the fecal suspension before the reaction and in the filtrate of the fecal suspension (flow-through) after the reaction, when the filtrate of the fecal suspension obtained by filtration with a PVDF filter having a pore size of 0.45 μm (A), an MCE filter having a pore size of 0.8 μm (B), an MCE filter having a pore size of 1.2 μm (C), or a PVDF filter having a pore size of 5.0 μm (D) was reacted with the capturing agent.
FIG. 5 is a diagram illustrating results of a Western blot for LPS/Lipid A in a culture supernatant obtained when predetermined bacteria are cultured, in a culture supernatant in which no bacteria are cultured, or in an eluate obtained

by reacting these supernatants with a capturing agent and eluting membrane vesicles captured by the capturing agent.

FIG. 6 is a diagram illustrating results of analysis of bacterial species profiles in feces, or in samples obtained when respective purification methods (ultracentrifugation purification and capturing agent purification) are performed on feces.

**Description of Embodiments**

Hereinafter, an embodiment of the present invention will be described in detail. However, the present invention is not limited to the following embodiment.

A method for purifying extracellular vesicles and/or membrane vesicles in feces according to the present embodiment (hereinafter also referred to as "the method according to the present embodiment") includes a process of filtering a sample of the feces with a filter (filtration process) and a process of bringing the filtered sample into contact with a capturing agent (contact process), in which the capturing agent contains a crosslinked polymer including monomer units having an acidic group and/or monomer units having a neutralizing base of the acidic group, and satisfies the predetermined conditions (i) and/or (ii).

In the contact process, extracellular vesicles and/or membrane vesicles are purified by extracellular vesicles and/or membrane vesicles being specifically captured by the capturing agent. Here, "capture" means that the extracellular vesicles and/or membrane vesicles specifically bind to the surface of the capturing agent by a physical interaction such as electrostatic attraction. The extracellular vesicles and/or membrane vesicles captured by the capturing agent may be used as they are (for example, for quantification or analysis of their contents), but may also be used after being separated from the capturing agent. Therefore, the method according to the present embodiment may further include a process of separating the extracellular vesicles and/or membrane vesicles from the capturing agent (separation process or recovery process).

In the present specification, "extracellular vesicles" refers to vesicles having a lipid bilayer, containing intracellular components, and secreted from cells into the extracellular space, and refers to those secreted from cells of organisms other than bacteria. The extracellular vesicles contain nucleic acids such as microRNA, messenger RNA (mRNA), and DNA; proteins such as growth factors and enzymes; lipids; and the like, and are involved in many important functions in vivo, such as intercellular communication, regulation of gene transcription rates, and induction and modulation of immune responses. Examples of the extracellular vesicles include exosomes, microvesicles, apoptotic bodies, and the like.

As the organisms other than bacteria, any organism from which feces can be collected may be used, and examples include mammals, birds, reptiles, or fish, and mammals are preferred. Examples of mammals include humans, chimpanzees, mice, hamsters, cats, dogs, cattle, horses, rats, and the like.

In the present specification, "membrane vesicles (MV)" refers to vesicles containing components inside bacteria and secreted by bacteria. Membrane vesicles contain nucleic acids such as DNA; pathogenic toxins; signaling proteins; proteins such as enzymes; lipids; polysaccharides; peptidoglycans; and the like, and are involved in various functions at their destinations, such as interactions between microorganisms, transmission of pathogenicity, transformation, survival under stress conditions, and inflammation control. The membrane vesicles to which the method according to the present embodiment is applied are preferably those secreted by bacteria present in the intestinal tract, particularly in the large intestine (intestinal bacteria).

The bacteria may be Gram-negative bacteria or Gram-positive bacteria. Membrane vesicles secreted by Gram-negative bacteria are also referred to as outer membrane vesicles (OMV).

Examples of Gram-negative bacteria include bacteria belonging to the genus Bacteroides, the genus Escherichia, the genus Salmonella, or the genus Pseudomonas. Examples of bacteria belonging to the genus Bacteroides include Bacteroides vulgatus. Examples of bacteria belonging to the genus Escherichia include Escherichia coli. Examples of bacteria belonging to the genus Salmonella include Salmonella enterica. Examples of bacteria belonging to the genus Pseudomonas include Pseudomonas aeruginosa.

Examples of Gram-positive bacteria include bacteria belonging to the genus Lactobacillus, the genus Bacillus, the genus Streptococcus, or the genus Clostridium. Examples of bacteria belonging to the genus Lactobacillus include Lactobacillus acidophilus. Examples of bacteria belonging to the genus Bacillus include Bacillus cereus. Examples of bacteria belonging to the genus Streptococcus include Streptococcus thermophilus. Examples of bacteria belonging to the genus Clostridium include Clostridium innocuum.

Examples of bacteria include bacteria belonging to the genus Prevotella, the genus Clostridia UCG-014, the genus Alistipes, the genus Bacteroides, the genus Faecalibacterium, the genus Barnesiella, the genus Turicibacter, the genus Romboutsia, Prevotella copri, Alistipes inops, bacteria belonging to the family Oscillospiraceae, Alistipes shahii, Clostridium leptum, Alistipes indistinctus, Bacteroides coprocola, bacteria belonging to the genus Subdoligranulum, bacteria belonging to the family Ruminococcaceae, and the like.

In the present specification, "a sample of feces" refers to a sample containing feces, and may be one prepared

using feces for implementing the filtration process described later. Examples of samples of feces include a fecal suspension obtained by suspending feces in an aqueous medium, and a supernatant of the fecal suspension, which may contain the extracellular vesicles and/or membrane vesicles. Examples of the aqueous medium include physiological saline such as phosphate-buffered physiological saline (PBS), sterilized water, and pH buffer solutions such as Good's buffers. The supernatant of the fecal suspension can be obtained by centrifuging the fecal suspension to remove solid matter.

The filtration process is a process of filtering a sample of the feces with a filter to obtain a filtrate. By including the filtration process, the method according to the present embodiment improves the capture efficiency of extracellular vesicles and/or membrane vesicles in the contact process described later.

From the viewpoint of further improving the capture efficiency of extracellular vesicles and/or membrane vesicles in the contact process described later, the pore size of the filter in the filtration process may be, for example, 5 μm or less, 4 μm or less, 3 μm or less, 2 μm or less, 1 μm or less, 0.8 μm or less, 0.7 μm or less, 0.6 μm or less, 0.5 μm or less, or 0.45 μm or less. The pore size of the filter in the filtration process may also be, for example, 0.2 μm or more, or 0.3 μm or more.

Examples of materials for the filter used in the filtration process include, without particular limitation, polyvinylidene fluoride (PVDF), mixed cellulose ester (a mixture of cellulose acetate and nitrocellulose; MCE), polyethersulfone (PES), and glass fiber.

In the contact process, the fecal sample having undergone the filtration process is brought into contact with the capturing agent, thereby causing extracellular vesicles and/or membrane vesicles in the feces and the capturing agent to react with each other, and capturing extracellular vesicles and/or membrane vesicles with the capturing agent. The contacting of the fecal sample that has undergone the filtration process with the capturing agent may be performed by mixing the two.

The capturing agent in the contact process contains a crosslinked polymer including monomer units having an acidic group and/or monomer units having a neutralizing base of the acidic group, and the capturing agent satisfies the following (i) and/or (ii):

(i) the crosslinked polymer includes at least monomer units having a neutralizing base between the acidic group and a first cationic surfactant; and
(ii) the capturing agent further contains a second cationic surfactant (provided that the second cationic surfactant is excluded when the second cationic surfactant is a crosslinked polymer including monomer units having a neutralizing base of the acidic group).

When the capturing agent satisfies only (i), the capturing agent contains both monomer units having an acidic group and monomer units having a neutralizing base between the acidic group and the first cationic surfactant, or contains only a crosslinked polymer including monomer units having a neutralizing base between the acidic group and the first cationic surfactant. That is, when the capturing agent satisfies only (i), the capturing agent does not contain the first cationic surfactant that is not forming a neutralizing base with the acidic group. In addition, when the capturing agent satisfies only (ii), the expression "the capturing agent further contains a second cationic surfactant (provided that the second cationic surfactant is excluded when the second cationic surfactant is a crosslinked polymer including monomer units having a neutralizing base of the acidic group)" means that the second cationic surfactant is present in the capturing agent in a state not reacted with the crosslinked polymer, that is, that the second cationic surfactant does not form a neutralizing base with the acidic group possessed by the crosslinked polymer. That is, when the capturing agent satisfies only (ii), the crosslinked polymer contained in the capturing agent does not contain a monomer unit having a neutralizing base with the second cationic surfactant. In this case, the second cationic surfactant is not a crosslinked polymer including monomer units having a neutralizing base of the acidic group. When the capturing agent satisfies both (i) and (ii), the capturing agent may contain a crosslinked polymer including monomer units having an acidic group, and monomer units having a neutralizing base between the acidic group and the first cationic surfactant, and a second cationic surfactant that is not forming a neutralizing base with the acidic group possessed by the crosslinked polymer; and may contain a crosslinked polymer including monomer units having a neutralizing base between the acidic group and the first cationic surfactant, and a second cationic surfactant that is not forming a neutralizing base with the acidic group possessed by the crosslinked polymer. The types of the first cationic surfactant in (i) and the second cationic surfactant in (ii) may be the same or different.

The term "acidic group" refers to a functional group that releases a proton ($H^+$) in an aqueous solution to exhibit acidity (pH<7. 0). Although not particularly limited, acidic groups are preferably organic acid groups, and examples include carboxyl groups (-COOH), sulfonic acid groups [$-S(=O)_2(-OH)$], sulfuric acid groups [$-O-S(=O)_2(-OH)$], phosphonic acid groups [$-P(=O)(-OH)_2$], and phosphoric acid groups [-O-P(=O)(-OH)].

The neutralizing base of the acidic group refers to a functional group formed as a result of a reaction between the acidic group and any compound having a cationic group capable of undergoing a neutralization reaction with the acidic group, in which a cation derived from the compound having the cationic group ionically bonds to the acidic group in place of the proton ($H^+$) of the acidic group. For example, in a case where the acidic group is a carboxyl group (-COOH) and the

cation derived from a compound having a cationic group is $XY^+$ (where $Y^+$ is a cationic group and X is any group covalently bonded to $Y^+$), the neutralizing base is -COOYX. Specific examples of cations include metals (such as monovalent or divalent metals), and cations represented by formula $NR_4^+$ (wherein each R independently represents a hydrogen atom or a hydrocarbon group that may be substituted, and two or three Rs may form a ring together with the nitrogen atom to which they are adjacent). In addition, the cation may also be a cation derived from a cationic surfactant. The "cationic surfactant" is a surfactant having a cationic group. In the present specification, the term "cationic group" encompasses a group that itself forms a cation, and a group that itself does not form a cation but is capable of forming a cation by binding a proton. The neutralizing base between the acidic group and a cationic surfactant refers to a functional group (acidic group + cationic surfactant residue) formed from a reaction between the acidic group and the cationic surfactant via the cationic group of the cationic surfactant.

Examples of monovalent metals include alkali metals such as lithium, sodium, and potassium. Examples of divalent metals include alkaline earth metals such as magnesium, calcium, and barium; lead; zinc; and tin.

In the cation represented by the formula: $NR_4^+$, when R is a hydrocarbon group that may be substituted, the hydrocarbon group may be, for example, an alkyl group, a cycloalkyl group, an aryl group, or an aralkyl group. Examples of alkyl groups include $C_{1-6}$ alkyl groups such as methyl, ethyl, propyl (n-propyl or isopropyl), and butyl (n-butyl, isobutyl, sec-butyl, or t-butyl). Examples of cycloalkyl groups include $C_{5-14}$ cycloalkyl groups such as cyclopentyl and cyclohexyl. Examples of aryl groups include $C_{6-14}$ aryl groups such as phenyl and naphthyl. Examples of aralkyl groups include $C_{7-14}$ aralkyl groups such as benzyl and phenethyl. The cation represented by the formula: $NR_4^+$ may be a cation derived from a cationic surfactant or may be a cation not derived from a cationic surfactant.

Examples of substituents that may substitute the hydrocarbon group include halogen atoms, hydroxyl groups, and mercapto groups.

In the cation represented by the formula: $NR_4^+$, in a case where two or three Rs form a ring together with the nitrogen atom to which they are adjacent, the ring may be a monocyclic ring such as a pyridine ring or an imidazole ring, or may be a fused ring such as a quinoline ring. The ring may have one or more substituents, and examples of the substituent include halogen atoms, hydroxyl groups, amino groups, alkyl groups, haloalkyl groups, hydroxyalkyl groups, and N,N-dialkylamino groups. The number of substituents may be, for example, one, two, or three.

It is preferable that the cationic surfactant has a cationic group and a hydrocarbon group having 2 to 30 carbon atoms, the hydrocarbon group being optionally substituted, and it is more preferable that the cationic surfactant has a cationic group and a hydrocarbon group having 12 to 30 carbon atoms, the hydrocarbon group being optionally substituted. The number of the hydrocarbon groups and the cationic groups in the cationic surfactant may be one or two or more.

The hydrocarbon group having 12 to 30 carbon atoms may be any of an aliphatic hydrocarbon group such as an alkyl group or an alkenyl group, a cyclic hydrocarbon group such as a cycloalkyl group or a cycloalkenyl group, or an aromatic hydrocarbon group; however, it is preferably an aliphatic hydrocarbon group. Examples of substituents of the hydrocarbon group include hydroxyl groups, oxo groups (=O), alkoxy groups, alkenyloxy groups, and combinations thereof. The number of the substituents of the hydrocarbon group may be, for example, one, two, three, four, or five.

Examples of the aliphatic hydrocarbon group having 12 to 30 carbon atoms include saturated or unsaturated hydrocarbon groups, such as lauryl, myristyl, palmityl, stearyl, oleyl, linoleyl, and arachidyl groups, which are aliphatic hydrocarbon groups having 12 to 20 carbon atoms.

For example, hexadecyltrimethylammonium chloride has a palmityl group as an aliphatic hydrocarbon group having 12 to 30 carbon atoms, the aliphatic hydrocarbon group being optionally substituted.

Examples of the cyclic hydrocarbon group having 12 to 30 carbon atoms include groups having a steroid skeleton. The steroid skeleton is represented, for example, by Formula (1) below:

[Chem. 1]

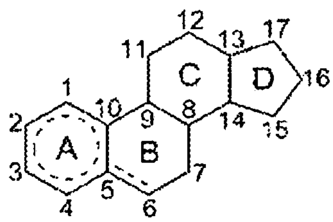

(1)

(wherein the broken line of ring A indicates that an arbitrary carbon-carbon single bond may be a carbon-carbon double bond, the broken line of ring B indicates that the carbon-carbon single bond may be a carbon-carbon double bond, and

rings A to D may each be substituted. The numbers on the rings are provided merely for convenience to indicate the positions of substituents.)

Examples of the substituents that may substitute rings A to D include hydroxyl groups, oxo groups (=O), alkyl groups (for example, alkyl groups having 1 to 4 carbon atoms such as methyl), and combinations thereof (for example, hydroxyalkyl groups). Examples of the substitution positions for the substituent are not particularly limited; however, it includes the 3-position, 7-position, 10-position, 11-position, 12-position, 13-position, 17-position, and the like.

The skeleton represented by Formula (1) includes the skeletons represented by Formulas (1-1) to (1-5):

[Chem. 2]

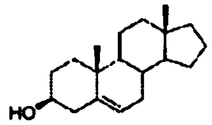

(1-1)

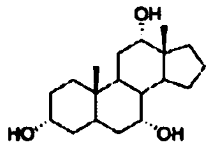

(1-2)

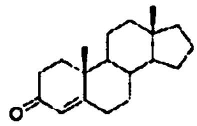

(1-3)

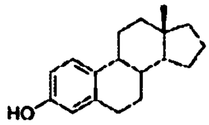

(1-4)

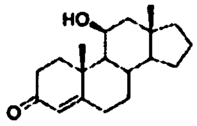

(1-5)

The cyclic hydrocarbon group having 12 to 30 carbon atoms is preferably a cyclic hydrocarbon group having 20 to 30 carbon atoms, more preferably a group in which an alkylene group having 2 to 4 carbon atoms (for example, ethylene or propylene) that may be substituted is linked to the 17-position of the skeleton represented by Formula (1), and even more preferably a group in which an alkylene group having 2 to 4 carbon atoms (for example, ethylene or propylene) that may be substituted is linked to the 17-position of any of the skeletons represented by Formulas (1-1) to (1-5).

Examples of the aromatic hydrocarbon group having 12 to 30 carbon atoms include fluorene, anthracene, phenanthrene, tetracene, pyrene, triphenylene, chrysene, and tetraphenylene.

The hydrocarbon group having 12 to 30 carbon atoms and being optionally substituted may be a group having a di-$C_{12-30}$ acylglycerol skeleton or a group having a steroid skeleton.

Examples of the cationic group in the cationic surfactant include $-N(R^1)_2$ (wherein each $R^1$ independently represents a hydrogen atom or a hydrocarbon group having 1 to 6 carbon atoms, the hydrocarbon group being optionally substituted) and $-N+(R^2)_3$ (wherein each $R^2$ independently represents a hydrogen atom or a hydrocarbon group having 1 to 6 carbon atoms, the hydrocarbon group being optionally substituted), and $-N+(R^2)_3$ is preferred.

Examples of the hydrocarbon group having 1 to 6 carbon atoms in $R^1$ and $R^2$ include alkyl groups such as methyl, ethyl, propyl, butyl, pentyl, and hexyl groups. Examples of substituents that may substitute the hydrocarbon group having 1 to 6 carbon atoms include hydroxyl groups and oxo groups.

The group represented by the above formula: $-N(R^1)_2$ is preferably $-NH_2$ or $-N(R^{11})_2$ (wherein $R^{11}$ represents a hydroxyalkyl group having 1 to 5 carbon atoms). Examples of the hydroxyalkyl group having 1 to 5 carbon atoms include hydroxyethyl, hydroxypropyl, hydroxybutyl, and hydroxypentyl groups (including pentahydroxypentyl groups).

Examples of the group represented by the above formula: $-N+(R^2)_3$ include $-N^+(CH_3)_3$, $-N^+(CH_3)_2(C_2H_5)$, and $-N^+(CH_3)_2(C_3H_7)$.

Examples of the cationic surfactant include compounds having an aliphatic hydrocarbon group having 12 to 30 carbon atoms and a cationic group (for example, hexadecyltrimethylammonium chloride), compounds having a cyclic hydrocarbon group having 12 to 30 carbon atoms and a cationic group, compounds having a cationic group (for example, triethanolamine), and polymers having a cationic group (for example, polylysine or polyethyleneimine). It is preferable that the cationic surfactant be a compound having an aliphatic hydrocarbon group having 12 to 30 carbon atoms and a cationic group, and it is more preferable that the cationic surfactant be hexadecyltrimethylammonium chloride (HDTMA-Cl).

The lower limit of the molar concentration of the cationic group per 1 mol of the cationic surfactant is preferably 1 mol or more, more preferably 5 mol or more, and even more preferably 10 mol or more. In addition, the upper limit of the molar concentration of the cationic group per 1 mol of the cationic surfactant is preferably 100 mol or less.

The lower limit of the formula weight or number average molecular weight of the cationic surfactant is preferably 300 or more, and more preferably 500 or more. The upper limit of the formula weight or number average molecular weight of the cationic surfactant is preferably 10,000 or less. The number average molecular weight of the cationic surfactant can be measured by gel permeation chromatography (GPC) under the following conditions.

<GPC Measurement Conditions>

[1] Instrument: Gel permeation chromatography (Model HLC-8120GPC, manufactured by Tosoh Corporation)
[2] Columns: "TSKgel G6000PWxl" and "TSKgel G3000PWxl" (both manufactured by Tosoh Corporation) connected in series
[3] Eluent: A solution prepared by dissolving 0.5 wt% sodium acetate in a methanol/water mixture (30/70, volume ratio)
[4] Standard substance: Polyethylene glycol (PEG)
[5] Injection conditions: Sample concentration 0.25 wt%, column temperature 40°C

Examples of monomers having an acidic group include unsaturated carboxylic acids. Examples of unsaturated carboxylic acids include unsaturated monocarboxylic acids and unsaturated dicarboxylic acids. Examples of unsaturated monocarboxylic acids include unsaturated monocarboxylic acids having 3 to 10 carbon atoms, such as acrylic acid, methacrylic acid, and crotonic acid, and acrylic acid is more preferred. Examples of unsaturated dicarboxylic acids (including anhydrides) include unsaturated dicarboxylic acids having 4 to 10 carbon atoms, such as maleic acid, fumaric acid, citraconic acid, and itaconic acid, and anhydrides thereof.
Other examples of monomers having an acidic group include (meth)acrylic monomers having a sulfonic acid group (for example, (meth)acrylic sulfoalkyl esters such as (meth)acrylic acid 2-sulfoethyl, and N-sulfoalkyl (meth) acrylamides such as 2-(meth)acrylamido-2-methylpropanesulfonic acid), and (meth)acrylic monomers having a phosphoric acid group (for example, (meth)acrylic phosphonooxyalkyl esters such as 2-((meth)acryloyloxy)ethyl phosphate). In the present specification, "(meth)acrylic" means acrylic and/or methacrylic, and "(meth)acryloyloxy" means acryloyloxy and/or methacryloyloxy.
The monomer having an acidic group may be a single type or a combination of two or more types.
Examples of monomers having a neutralizing base of the acidic group include unsaturated carboxylate salts. Examples of the unsaturated carboxylate salts include alkali metal salts (for example, sodium salts and potassium salts) of unsaturated carboxylic acids, alkylamine salts (for example, trialkylamine salts such as triethylamine salts), alkanolamine salts (for example, dialkanolamine salts such as diethanolamine salts, and trialkanolamine salts such as triethanolamine salts), ammonium salts, and tetraalkylammonium salts (for example, tetramethylammonium salts and tetraethylammonium salts). In addition, examples of the unsaturated carboxylate salts include unsaturated monocarboxylate salts and unsaturated dicarboxylate salts. Examples of the unsaturated monocarboxylate salts include unsaturated monocarboxylate salts having 3 to 10 carbon atoms, such as acrylate salts, methacrylate salts, and crotonate salts, and acrylate salts are more preferred. Examples of unsaturated dicarboxylate salts (including anhydride salts) include unsaturated dicarboxylate salts having 4 to 10 carbon atoms, preferably maleate salts, fumarate salts, citraconate salts, and itaconate salts, and anhydride salts thereof.
Other examples of monomers having a neutralizing base of the acidic group include neutralization salts of the above (meth)acrylic monomers having a sulfonic acid group, and neutralization salts of the above (meth)acrylic monomers having a phosphoric acid group. More specific examples of such neutralization salts include the above alkali metal salts.
The monomer having a neutralizing base of the acidic group may be a single type or a combination of two or more types.
It is preferable that the crosslinked polymer contain both monomer units having an acidic group and monomer units having a neutralizing base of the acidic group. That is, in the crosslinked polymer containing monomer units having an acidic group, a portion of the monomer units having an acidic group is neutralized and replaced with monomer units having a neutralizing base of the acidic group. The degree of neutralization [100 x (the number of moles of the neutralizing base of the acidic group)/(the number of total moles of the acidic group and the neutralizing base of the acidic group)] is not particularly limited; however, it may be, for example, 25 mole% or more, 30 mole% or more, 35 mole% or more, 40 mole% or more, 45 mole% or more, or 50 mole% or more, and it may be 90 mole% or less, 85 mole% or less, or 80 mole% or less.
In addition to monomer units having an acidic group and/or monomer units having a neutralizing base of the acidic group, the crosslinked polymer may further include other monomer units different from these monomers. Examples of the other monomers include the following mono-functional ethylenically unsaturated monomers:

· Esters of unsaturated carboxylic acids (for example, acrylic acid, methacrylic acid, etc.) (for example, alkyl esters such as methyl ester, ethyl ester, propyl ester, butyl ester, pentyl ester, hexyl ester, heptyl ester, octyl ester, and 2-ethylhexyl ester; hydroxyalkyl esters such as 2-hydroxyethyl ester; haloalkyl esters such as 2,2,2-trifluoroethyl ester; aminoalkyl esters such as 2-aminoethyl ester; (mono- or dialkylamino)alkyl esters such as 2-(N,N-dimethylamino) ethyl ester; cycloalkyl esters such as cyclohexyl ester; aryl esters such as phenyl ester and naphthyl ester; aralkyl esters such as benzyl ester and phenethyl ester; glycidyl esters; and polyethylene glycol esters, etc.) · Unsaturated carboxylic acid amides (for example, free amides; N-monoalkyl amides, N,N-dialkyl amides, and other N-substituted

amides, etc.)
· Unsaturated dicarboxylic acid imides (for example, maleimide, citraconimide, itaconimide, and their N-alkyl, N-cycloalkyl, or N-aryl substituted derivatives)
· Alkenyl esters of saturated carboxylic acids (for example, vinyl esters or allyl esters of acetic acid or propionic acid)
· Esters or amides of unsaturated sulfonic acids (for example, esters or amides corresponding to the above unsaturated carboxylic acid esters or amides)
· Unsaturated alcohols (for example, allyl alcohol, propenyl alcohol) · Unsaturated ethers (for example, alkyl vinyl ethers such as methyl vinyl ether or ethyl vinyl ether; alkyl allyl ethers such as methyl allyl ether or ethyl allyl ether; cycloalkyl vinyl ethers such as cyclohexyl vinyl ether; glycidyl vinyl ether)
· Unsaturated nitriles (for example, acrylonitrile, methacrylonitrile) · Olefins (for example, ethylene, propylene, butene, pentene, hexene) · Aromatic vinyl compounds (for example, styrene, $\alpha$-methylstyrene, vinyltoluene, hydroxystyrene)
· Heterocyclic vinyl compounds (for example, N-vinylpyrrolidone)

In a case where the crosslinked polymer further contains another monomer different from the monomer having an acidic group and/or the monomer having a neutralizing base of the acidic group, the other monomer may be used alone as a single species or may be used as a combination of two or more monomers.
It is preferable that the monomers constituting the crosslinked polymer have a solubility of 1 g or more (g/100 g of water) in water at 25°C, and it is more preferable that they have a solubility of 5 g or more. It is also preferable that the monomers constituting the crosslinked polymer be water-miscible.
The crosslinked polymer is a polymer having a crosslinked structure in which constituent atoms of a plurality of linear polymers (polymers of monomers having acidic groups and/or monomers having neutralizing bases of the acidic groups) are covalently bonded directly or through other atoms. In a case where the monomers constituting the crosslinked polymer have reactive groups (for example, a combination of a monomer having a carboxyl group and a monomer having an amino group), self-crosslinking may be performed, but crosslinking with any desired crosslinking agent may be performed as necessary.
Examples of methods for crosslinking using a crosslinking agent include the following:

(1) A method in which the above monomers and an internal crosslinking agent are subjected to a polymerization reaction to directly obtain a crosslinked polymer having a crosslinked structure (for example, methods described in Japanese Unexamined Patent Publication No. 2003-225565 and Japanese Unexamined Patent Publication No. 2005-075982).
(2) A method in which a polymer containing the above monomers as essential monomer units is crosslinked using a surface crosslinking agent to obtain a crosslinked polymer (for example, methods described in Japanese Patent No. 3648553, Japanese Unexamined Patent Publication No. 2003-165883, Japanese Unexamined Patent Publication No. 2003-225565, Japanese Unexamined Patent Publication No. 2005-75982, and Japanese Unexamined Patent Publication No. 2005-95759).

The crosslinking agent (including internal crosslinking agents and surface crosslinking agents) is not particularly limited, and examples thereof include, for example, difunctional or higher ethylenically unsaturated monomers listed below.

- Alkenyl ethers (for example, vinyl ethers, allyl ethers, and the like) of divalent or higher alcohols (for example, alkylene glycols or polyalkylene glycols such as ethylene glycol, polyethylene glycol, propylene glycol, butylene glycol, and neopentyl glycol (the preferable number of carbon atoms in the alkylene group is 2 to 4, and the preferable repetition number of the alkylene oxide is 10 to 50), glycerin, polyglycerin, trimethylolethane, trimethylolpropane, pentaerythritol, dipentaerythritol, and sorbitan)

· Esters of unsaturated carboxylic acids (for example, acrylic acid, methacrylic acid, and the like) with divalent or higher alcohols (for example, those exemplified in the above ethers)
· Alkenyl esters of unsaturated carboxylic acids (for example, acrylic acid, methacrylic acid, and the like) (for example, vinyl esters, allyl esters, and the like)
· Alkenyl esters of polycarboxylic acids (for example, tartaric acid, citric acid, adipic acid, and the like) (for example, vinyl esters, allyl esters, and the like)
· Alkenyl esters of isocyanuric acid (for example, triallyl isocyanurate and the like)
· Alkylene bisacrylamides (for example, methylene bisacrylamide and the like)
Alkylene bismethacrylamides (for example, methylene bismethacrylamide and the like)
· Di- or trialkenylamines (for example, diallylamine, triallylamine and the like)
· Aromatic polyvinyl compounds (for example, divinylbenzene and the like)

It is also possible to use a crosslinking agent having at least two functional groups capable of reacting with the substituents (carboxyl groups, hydroxyl groups, or the like) of the monomers. Examples of such crosslinking agents include polyvalent alcohol glycidyl ethers (for example, alkylene glycol diglycidyl ethers such as ethylene glycol diglycidyl ether, and alkanetriol di- or triglycidyl ethers such as glycerin diglycidyl ether).

The crosslinking agent may be used alone as a single species or as a combination of two or more species.

It is preferable that the amount of the crosslinking agent be appropriately selected depending on the desired crosslinking density, for example, the amount with respect to the monomers constituting the crosslinked polymer may be 0.005 mol% or more or 0.01 mol% or more, and may be 0.5 mol% or less or 0.4 mol% or less.

In a case where the capturing agent satisfies (ii), the second cationic surfactant may be the cationic surfactant described above (provided that the second cationic surfactant is not a crosslinked polymer containing monomer units having a neutralizing base of the acidic group). In a case where the "cationic group" possessed by the second cationic surfactant is a "group that itself forms a cation", examples of the counter anion include halide ions such as $F^-$, $Cl^-$, $Br^-$, and $I^-$.

Whether the capturing agent satisfies (i) and/or (ii) can be identified, for example, by immersing the capturing agent in an appropriate solvent (for example, hydrocarbon solvents such as hexane), releasing the cationic surfactant into the solvent, isolating the cationic surfactant, and subjecting it to NMR and gas chromatography.

It is preferable that the capturing agent be particles having a volume-average particle diameter within a range of 50 μm or more, 60 μm or more, 70 μm or more, 80 μm or more, 90 μm or more, 100 μm or more, 110 μm or more, 120 μm or more, 130 μm or more, 140 μm or more, or 150 μm or more. It is also preferable that the capturing agent be particles having a volume-average particle diameter within a range of 2000 μm or less, 1500 μm or less, 1000 μm or less, 500 μm or less, or 300 μm or less. The volume-average particle diameter can be measured using the method described in the examples below.

It is preferable that the water absorption ratio (the absorption ratio in physiological saline) of the capturing agent of the present invention in physiological saline be 1 g/g or more, more preferably 3 g/g or more, still more preferably 5 g/g or more, even more preferably 10 g/g or more, and most preferably 15 g/g or more. In a case where the water absorption ratio (the absorption ratio in physiological saline) of the capturing agent in physiological saline is too low, there is a tendency that the ratio increases by increasing the amount of the above crosslinking agent. The upper limit of the water absorption ratio (the absorption ratio in physiological saline) of the capturing agent in physiological saline is not particularly limited; however, it is preferably 120 g/g or less, more preferably 100 g/g or less, and particularly preferably 50 g/g or less. The absorption ratio in physiological saline can be measured, for example, by the method described in the examples below.

It is preferable that, in a case where the capturing agent satisfies (i) and/or (ii), the molar concentration of the cationic group in the capturing agent be from $6 \times 10^{-6}$ mol/g to $1 \times 10^{-4}$ mol/g, based on the weight of the capturing agent after drying. The weight of the capturing agent after drying can be measured, for example, by the following method. 1 g of the capturing agent is placed on a dish, covered with a filter paper, and heat-dried in a forced-circulation drying oven at 130°C for 60 minutes, and the weight of the residue after the drying can be regarded as the weight of the crosslinked polymer after drying.

The capturing agent of the present invention can be produced, for example, through the production of a crosslinked polymer as follows. The crosslinked polymer in the present invention can be produced by a method including a process of polymerizing (solution polymerization, emulsion polymerization, suspension polymerization, etc.) a composition containing a monomer, a crosslinking agent, a solvent, an initiator, and optionally a neutralizing agent, a process of drying the polymer, and optionally a process of classifying the polymer. The capturing agent of the present invention can be produced, for example, by a method including a process of mixing the above crosslinked polymer with a cationic surfactant (preferably mixing for 1 to 2 minutes at 20 to 30°C), and optionally a process of aging the mixture at 100 to 150°C for 10 to 60 minutes.

In the contact process, the amount of the capturing agent to be brought into contact with the fecal sample that has undergone the filtration process is preferably, with respect to 1 g of feces used when preparing the fecal sample, for example, 0.01 mg or more, 0.05 mg or more, 0.1 mg or more, 0.5 mg or more, 1 mg or more, 5 mg or more, 10 mg or more, 15 mg or more, 20 mg or more, or 25 mg or more. In the contact process, the amount of the capturing agent to be brought into contact with the fecal sample that has undergone the filtration process is preferably, with respect to 1 g of feces used when preparing the fecal sample, for example, 100 mg or less, 90 mg or less, 80 mg or less, 70 mg or less, 60 mg or less, 50 mg or less, 40 mg or less, 30 mg or less, 20 mg or less, or 10 mg or less.

The contact process may include maintaining the fecal sample having undergone the filtration process in contact with the capturing agent for, for example, 10 minutes or more, 15 minutes or more, 20 minutes or more, 25 minutes or more, or 30 minutes or more. The contact process may include maintaining the fecal sample having undergone the filtration process in contact with the capturing agent for, for example, 6 hours or less, 5 hours or less, 4 hours or less, 3 hours or less, 2 hours or less, 1 hour or less, or 50 minutes or less. The contact process may be carried out, for example, at 4°C to 40°C, 10°C to 35°C, or 15°C to 30°C, and is not particularly limited.

The method according to the present embodiment may further include a process of recovering (recovery process) the extracellular vesicles and/or membrane vesicles captured by the capturing agent through the contact

process. In this recovery process, the extracellular vesicles and/or membrane vesicles captured by the capturing agent are separated from the capturing agent. Accordingly, the recovery process is also referred to as the separation process. By including the recovery process, the method according to the present embodiment enables obtaining purified extracellular vesicles and/or membrane vesicles.

The recovery process can be performed by conventional methods, and, for example, after the contact process, the extracellular vesicles and/or membrane vesicles may be eluted from the capturing agent by changing the salt concentration or pH.

As described above, according to the method of the present embodiment, extracellular vesicles and/or membrane vesicles in feces can be purified by carrying out the filtration process on the fecal sample and then carrying out the contact process. Accordingly, an embodiment of the present invention also provides a kit for purifying extracellular vesicles and/or membrane vesicles in feces, the kit including a filter and the capturing agent described above.

[Example]

The present invention will be described in more detail below based on examples. However, the present invention is not limited thereto.

<Production of Capturing Agent>

In this Example, the capturing agent of the present invention was produced according to the following procedure. First, acrylic acid 116. 5 g, ion-exchanged water 272. 2 g, and ethylene glycol diglycidyl ether 0.58 g (0.5 wt% / acrylic acid) as a crosslinking agent were placed in a 1-liter beaker, mixed, and the crosslinking agent was dissolved. While cooling the beaker in an ice bath, 96.1 g of a 48. 5 wt% aqueous sodium hydroxide solution was added, and part of the acrylic acid (72 mol%) was neutralized. After the neutralized monomer solution was cooled to 5°C, 9. 3 g of a 2 wt% aqueous potassium persulfate solution was added as a polymerization initiator to prepare a monomer aqueous solution. A 2-liter separable flask equipped with a stirrer and a condenser (cooler) was charged with cyclohexane 1434 g and 7.1 g of Reodol SP-S10V (manufactured by Kao Corporation, sorbitan monostearate) as a dispersant, heated to an internal temperature of 60°C using a hot-water bath, and stirred to dissolve the dispersant in the cyclohexane. Nitrogen was passed through the solution in the separable flask to reduce the dissolved oxygen in the cyclohexane to 0.1 ppm or less, after which, while stirring using the stirrer, 350 g of the monomer aqueous solution was added dropwise using a dropping funnel. Reverse-phase suspension polymerization was carried out at a polymerization temperature of 80°C, and after completion of the dropwise addition of the monomer aqueous solution, the mixture was further heated for 2 hours to complete the suspension polymerization, thereby obtaining spherical hydrogel beads in cyclohexane. After stopping the rotation of the stirrer, the resulting hydrogel beads were allowed to settle, and the cyclohexane was removed by decantation. The remaining hydrogel beads were washed several times with cyclohexane to remove the dispersant adhering to the hydrogel. The obtained spherical hydrogel beads were spread on release paper and dried for 1 hour in a vacuum dryer at 130°C (degree of vacuum: 10,000 to 20,000 Pa) to obtain a crosslinked polymer A.

Next, the crosslinked polymer A was adjusted to a particle size of 300 to 500 $\mu$m by using sieves having openings of 300 $\mu$m and 500 $\mu$m, thereby obtaining a crosslinked polymer B.

Subsequently, 20 g of a 50 wt% potassium carbonate aqueous solution was sprayed onto 50 g of the crosslinked polymer B from a spray nozzle, and, after uniformly mixing, the mixture was heated at 130°C for 30 minutes and then cooled to room temperature to obtain crosslinked polymer C.

Finally, 1 g of a 5 wt% hexadecyltrimethylammonium chloride (HDTMA-Cl, from FUJIFILM Wako Pure Chemical Corporation) cyclohexane solution was added dropwise with a dropper to 10 g of the crosslinked polymer C, and, after uniformly mixing, the mixture was heated at 130°C for 30 minutes and then cooled to room temperature to obtain the capturing agent.

When the volume-average particle diameter of the capturing agent was measured with a particle analyzer (CAMSIZER XT, from Retsch GmbH), it was 420 $\mu$m.

The absorption ratio of physiological saline of the capturing agent was measured as follows. First, 1 g of a measurement sample was placed in a tea bag (20 cm in length and 10 cm in width) prepared using a nylon mesh having an opening of 63 $\mu$m (JIS Z8801-1:2006), and the tea bag was immersed in 1000 mL of physiological saline (0.9 wt% salt concentration) for 1 hour without stirring. Thereafter, the tea bag was hung for 15 minutes to drain off the liquid. Subsequently, the weight (h1) of the tea bag was measured, and the absorption ratio of physiological saline was determined from the following equation. The temperature of the physiological saline used and the measurement atmosphere was set to 25°C$\pm$2°C. Further, except that no measurement sample was used, the weight of the tea bag was measured in the same manner as described above, and this weight was defined as (h2).

$$\text{Absorption ratio of physiological saline (g/g)}=(h1)-(h2)$$

As a result, the absorption ratio of physiological saline of the capturing agent was 32 g/g, and the measured pH of the physiological saline solution was 7.0.The pH measurement was performed in accordance with the method described later. In addition, the weight ratio after heat-drying with respect to the weight of the capturing agent was 97.4%, and the molar concentration of the cationic groups with respect to the weight of the capturing agent after drying was $1.60\times10^{-5}$ mol/g.

<Physiological Saline Solution pH Measurement Method>

In a cylindrical 100-mL beaker having a diameter of 50 mm, physiological saline (0.9 wt% salt concentration) was added to 0.5 g of a measurement sample to make the total weight 100 g, and the mixture was stirred with a stirrer chip (length: 30 mm) at 60 rpm for 30 minutes at 25°C, followed by standing for 1 minute at 25°C. After standing, the pH of the supernatant at 25°C was measured with a pH meter, and the measured value was defined as the pH of the physiological saline solution.

[Reference Example 1: Evaluation of Exosome Capture from Urine or Feces using Capturing Agent]

<Comparative Example 1>

(1. Reaction between the urine supernatant and the capturing agent)

A 1.1-mL human urine sample was centrifuged at 500×g for 10 minutes, and 1 mL of the supernatant was collected. Then, the collected 1 mL of supernatant was added to a Micro Bio-Spin chromatography column (Bio-Rad, 7326204) packed with 40 mg of the capturing agent, and the mixture was allowed to stand at room temperature for 30 minutes. Thereafter, the column was centrifuged at 1,000×g for 1 minute to obtain the urine supernatant (flow-through) after reaction with the capturing agent.

(2. Measurement of the amount of exosomes)

The amount of exosomes captured by the capturing agent was evaluated by measuring the amount of exosomes in the urine supernatant before reaction with the capturing agent and in the urine supernatant (flow-through) after the reaction. The amount of exosomes was measured by a sandwich immunoassay using antibodies against CD9 and CD63, representative surface antigens of extracellular vesicles. The sandwich immunological assay was carried out by constructing a BLEIA measurement system based on bioluminescence and using a fully automatic analyzer BLEIA (registered trademark)-1200 (manufactured by Eiken Chemical Co., Ltd.) (bioluminescent enzyme immunoassay BLEIA method). As the primary reaction, magnetic particles on which an anti-human CD9 antibody (Cosmo Bio, SHI-EXO-M01) was immobilized, 50 μL of the sample, and a biotin-labeled anti-human CD63 antibody (Cosmo Bio, SHI-EXO-M02-B) were mixed and reacted at 37°C for 15 minutes. Thereafter, unreacted substances and the biotin-labeled antibody were removed by washing, and 80 μL of a Tris buffer containing streptavidin and biotin-labeled luciferase was added, followed by performing a secondary reaction at 37°C for an additional 15 minutes. After the secondary reaction, washing was performed again, 100 μL of a luminescent substrate solution (manufactured by Eiken Chemical Co., Ltd.) was added, and the luminescence signal was detected and quantified as a luminescence value.

<Comparative Example 2>

(1. Reaction between the supernatant of the fecal suspension and the capturing agent)

About 3 g of a human feces sample was suspended in 50 mL of PBS to obtain a fecal suspension. The fecal suspension was centrifuged at 3,000×g for 10 minutes, and the supernatant of the fecal suspension was collected. Subsequently, 1 mL of the collected supernatant was added to a micro Bio-Spin chromatography column (7326204, from Bio-Rad Laboratories, Inc.) packed with 40 mg of the capturing agent, and the mixture was allowed to stand at room temperature for 30 minutes. Thereafter, the column was centrifuged at 1,000×g for 1 minute to obtain the supernatant of the fecal suspension (flow-through) after the reaction with the capturing agent.

(2. Measurement of the amount of exosomes)

The measurement of the amount of exosomes captured by the capturing agent was performed as described above in "Measurement of the amount of exosomes".

<Results>

The results are shown in FIG. 1. FIG. 1(A) illustrates the CD9/CD63 luminescence values in the urine supernatant before the reaction with the capturing agent and in the flow-through. FIG. 1(B) illustrates the CD9/CD63 luminescence values in the supernatant of the fecal suspension before reacting the supernatant of the fecal suspension with the capturing agent, and in the flow-through.

As illustrated in FIG. 1(A), in a case where the urine supernatant was reacted with the capturing agent, the CD9/CD63 luminescence value in the flow-through was low, and it was found that the amount of exosomes in the flow-through was very low. That is, it was suggested that almost all exosomes in the urine supernatant were captured by the capturing agent. In addition, as illustrated in FIG. 1(B), in a case where the supernatant of the fecal suspension was reacted with the capturing agent, the flow-through exhibited a higher CD9/CD63 luminescence value compared to the supernatant of the fecal suspension before the reaction, and it was confirmed that the amount of exosomes in the flow-through was high. That is, it was suggested that the exosomes in the supernatant of the fecal suspension were not captured by the capturing agent.

Since the capturing agent is water-absorbent, the volume of the flow-through obtained after reacting the supernatant of the fecal suspension with the capturing agent decreases as compared with the supernatant of the fecal suspension before the reaction. Therefore, in a case where exosomes are not captured by the capturing agent, the exosomes in the flow-through become more concentrated than those in the supernatant of the fecal suspension before the reaction, and the CD9/CD63 luminescence value in the flow-through becomes higher than that in the supernatant of the fecal suspension before the reaction.

From the results of Reference Example 1, it was shown that exosomes in feces are not captured merely by reacting them with the capturing agent, and that extracellular vesicles in feces are more difficult to purify compared with those in urine.

[Test Example 1: Evaluation of Capturing of Exosomes in Feces by Filter Filtration and Capturing Agent]

<Example 1>

(1. Reaction between the filtrate of the supernatant of the fecal suspension and the capturing agent)

About 3 g of a human feces sample was suspended in 50 mL of PBS to obtain a fecal suspension. The fecal suspension was centrifuged at 3,000×g for 10 minutes, and the supernatant was collected. Subsequently, the collected supernatant was filtered through a PVDF filter having a pore size of 0.45 μm (manufactured by Whatman) to obtain a filtrate. The obtained filtrate was then added to a micro Bio-Spin chromatography column (7326204, from Bio-Rad Laboratories, Inc.) packed with 40 mg of the capturing agent, and allowed to stand at room temperature for 30 minutes. Thereafter, the column was centrifuged at 1,000×g for 1 minute to obtain the filtrate (flow-through) after the reaction with the capturing agent.

(2. Measurement of the amount of exosomes)

The measurement of the amount of exosomes was performed as described above in "Measurement of the amount of exosome".

<Comparative Example 3>

(1. Reaction between the supernatant of the fecal suspension and the capturing agent)

About 3 g of a human feces sample was suspended in 50 mL of PBS to obtain a fecal suspension. The fecal suspension was centrifuged at 3,000×g for 10 minutes, and the supernatant was collected. Thereafter, except that the collected supernatant was not filtered with a filter, the supernatant (flow-through) after the reaction with the capturing agent was obtained in the same manner as in Example 1.

(2. Measurement of the amount of exosomes)

The measurement of the amount of exosomes was performed as described above in "Measurement of the amount of exosome".

<Results>

The results are shown in FIG. 2. FIG. 2(A) illustrates the CD9/CD63 luminescence values in the supernatant of the fecal suspension before the reaction and in the flow-through in a case where the above supernatant was reacted with the capturing agent without being filtered with a filter. FIG. 2(B) illustrates the CD9/CD63 luminescence values in the supernatant of the fecal suspension before the reaction and in the flow-through in a case where the above supernatant was filtered with a filter and then reacted with the capturing agent.

As illustrated in FIG. 2(A), in a case where the supernatant of the fecal suspension was reacted with the capturing agent without being filtered with a filter, the amount of exosomes in the flow-through was higher than that in the supernatant of the fecal suspension before the reaction, suggesting that the exosomes in the supernatant of the fecal suspension were not captured by the capturing agent (FIG. 2(A)). In contrast, as illustrated in FIG. 2(B), in a case where the supernatant of the fecal suspension was filtered with a filter and then reacted with the capturing agent, the amount of exosomes in the flow-through was extremely low compared with that in the supernatant of the fecal suspension before the reaction, suggesting that the exosomes in the supernatant of the fecal suspension were captured by the capturing agent (FIG. 2(B)).

From the results of Test Example 1, it was shown that filtering the supernatant of the fecal suspension with a filter greatly improves the capturing efficiency of extracellular vesicles in the supernatant of the fecal suspension by the capturing agent.

[Reference Example 2: Evaluation of Capture Efficiency of Exosomes by Filter Filtration and Magnetic Beads]

As a purification method for extracellular vesicles, a method using the MagCapture (trademark) Exosome Isolation Kit PS (Fujifilm Wako Pure Chemical Corporation) is known. This method is excellent in both rapidity, enabling completion of purification of extracellular vesicles in about three hours, and recovery rate. The purification of extracellular vesicles from serum or feces was examined using the above method.

<Comparative Examples 4 and 5>

(1. Pretreatment)

1,200 μL of a human serum sample was centrifuged at 1,000×g for 10 minutes, and the resulting supernatant was used as the input for processing with the above kit. About 0.2 g of a human feces sample was suspended in 3.84 mL of Tris buffer to obtain a fecal suspension. The fecal suspension was centrifuged at 3,000×g for 10 minutes, and the supernatant was collected. Subsequently, the collected supernatant was filtered through a 0.45 μm filter (manufactured by Whatman), and the resulting filtrate was used as the input for processing with the above kit.

(2. Purification of exosomes)

For the purification of exosomes from the serum supernatant or from the filtrate of the supernatant of the fecal suspension, the MagCapture (trademark) Exosome Isolation Kit PS was used. The purification was performed in accordance with the attached instruction manual.

(3. Measurement of the amount of exosomes)

The amount of exosomes in the filtrate of the supernatant of the serum or the filtrate of the supernatant of the fecal suspension and the amount of exosomes in the sample residual liquid (flow-through) separated from the magnetic particles after reacting the filtrate of the supernatant of the serum or the filtrate of the supernatant of the fecal suspension with the magnetic particles for exosome capture of the above kit were measured, whereby the amount of exosomes captured by the kit was evaluated. The measurement of the amount of exosomes was performed as described above in "Measurement of the amount of exosome".

<Results>

The results are shown in FIG. 3. FIG. 3(A) illustrates the CD9/CD63 luminescence values in the serum

supernatant before processing and in the flow-through when the serum supernatant was processed using the MagCapture (trademark) Exosome Isolation Kit PS. FIG. 3(B) illustrates the CD9/CD63 luminescence values in the filtrate of the supernatant of the fecal suspension and in the flow-through in a case where the filtrate was processed with the same kit.

As illustrated in FIG. 3(A), in a case where the serum supernatant and the magnetic beads in the above-mentioned kit were reacted, the amount of exosomes in the flow-through was lower than that in the serum supernatant before processing, suggesting that the exosomes in the serum supernatant were captured by the magnetic beads of MagCapture. In contrast, as illustrated in FIG. 3(B), in a case where the filtrate of the fecal suspension supernatant and the magnetic beads in the above-mentioned kit were reacted, the amount of exosomes in the flow-through was higher than that in the filtrate, suggesting that the exosomes in the filtrate of the fecal suspension supernatant were not captured by the magnetic beads of MagCapture.

[Test Example 2: Evaluation of Filter Pore Size and Material in Capturing Exosomes in Feces by Filter Filtration and Capturing Agent]

<Examples 2 to 5>

(1. Reaction between supernatant of the fecal suspension and the capturing agent)

About 3 g of a human feces sample was suspended in 50 mL of PBS to obtain a fecal suspension. Subsequently, the fecal suspension was filtered through a PVDF filter having a pore size of 0.45 μm (manufactured by Whatman), an MCE filter having a pore size of 0.8 μm (manufactured by Millipore), an MCE filter having a pore size of 1.2 μm (Millipore), or a PVDF filter having a pore size of 5.0 μm (Millipore), respectively. The resulting filtrate was added to a micro Bio-Spin chromatography column (7326204, from Bio-Rad Laboratories, Inc.) packed with 40 mg of the capturing agent and allowed to stand at room temperature for 30 minutes. Thereafter, the column was centrifuged at 1,000×g for 1 minute to obtain the filtrate (flow-through) after the reaction with the capturing agent.

(2. Measurement of the amount of exosomes)

The measurement of the amount of exosomes was performed as described above in "Measurement of the amount of exosome".

<Results>

The results are shown in FIG. 4. FIGS. 4(A) to (D) respectively illustrate the results of calculating the CD9/CD63 luminescence values in the filtrate before the reaction and in the flow-through in a case where the filtrate filtered through the PVDF filter having a pore size of 0.45 μm, the MCE filter having a pore size of 0.8 μm, the MCE filter having a pore size of 1. 2 μm, or the PVDF filter having a pore size of 5. 0 μm was reacted with the capturing agent.

As illustrated in FIGS. 4(A) and 4(B), in a case where the fecal suspension was filtered with a filter having a pore size of 0.8 μm or less, almost no exosomes were present in the flow-through, suggesting that the exosomes in the fecal suspension were almost entirely captured by the capturing agent. On the other hand, as illustrated in FIGS. 4(C) and 4(D), in a case where the fecal suspension was filtered with a filter having a pore size of 1. 2 μm to 5. 0 μm, the presence of exosomes in the flow-through was observed. That is, it appeared that a portion of the exosomes in the supernatant of the fecal suspension passed through without being captured by the capturing agent. Furthermore, the material of the filter did not affect the capture of exosomes by the capturing agent.

[Test Example 3: Evaluation (1) of Capture of Outer Membrane Vesicles (OMVs) by Capturing Agent]

<Example 6>

(1. Culture of Bacteroides vulgatus)

Bacteroides vulgatus frozen and stored within Eiken Chemical Co., Ltd. was streaked onto Pourmedia sheep blood agar medium (manufactured by Eiken Chemical Co., Ltd.) and cultured overnight under anaerobic conditions at 37°C. Subsequently, bacteria were picked from colonies and inoculated into 100 mL of clinical thioglycolate medium "Eiken" (manufactured by Eiken Chemical Co., Ltd.), which had been dissolved and sterilized, and cultured overnight under anaerobic conditions at 37°C. As a control, medium without bacterial inoculation was cultured in the same manner. After cultivation, the supernatant was centrifuged at 3,000×g for 30 minutes and then further centrifuged at 40,000×g for 1.5 hours to remove bacterial cells.

(2. Reaction of the capturing agent with the culture supernatant)

1 mL of the supernatant of the medium cultured with bacteria or the supernatant of the medium not cultured with bacteria (medium supernatant_B. vulgatus or medium supernatant_control), obtained by centrifuging at 40,000×g, was added to a micro-biospin chromatography column (Bio-Rad, catalog No. 7326204) packed with 40 mg of the capturing agent, and allowed to stand at room temperature for 30 minutes. Thereafter, the column was centrifuged at 1,000×g for 1 minute to remove unreacted medium. Next, 500 μL of physiological saline was added and centrifuged at 1,000×g for 1 minute for removal, and this washing step was repeated three times. Then, 300 μL of a 0.5% Triton X-100 solution was added to the column and allowed to stand at room temperature for 10 minutes. The column was thereafter centrifuged at 1,000×g for 1 minute to obtain the eluate. This elution operation was repeated once more to obtain the eluate (eluate_B. vulgatus or eluate_control).

(3. Western blot analysis of the eluates)

Each eluate 17.5 μL was mixed with SDS and DTT and then heated at 95°C for 10 minutes to prepare a Western blot sample. The Western blot sample, 20 μL, was subjected to electrophoresis on an acrylamide gel having a concentration of 10 to 20%, transferred to a PVDF membrane, reacted with anti-Lipid A LPS antibody (manufactured by Invitrogen) overnight under refrigeration, subsequently reacted with HRP-labeled anti-goat antibody for 1 hour, then developed with ECL prime (GE Healthcare), and a specific band reacting with the anti-Lipid A LPS antibody was detected with iBright Imaging Systems (Thermo Fisher Scientific).

<Results>

The results are shown in FIG. 5. FIG. 5 is a diagram illustrating the results of the Western blot of LPS/Lipid A in the culture supernatant and in the eluate. As illustrated in FIG. 5, ladder-like bands, which appear to be LPS proteins, were detected in the eluate in the same manner as in the culture supernatant, suggesting that the capturing agent captured the membrane vesicles.

[Test Example 4: Evaluation (2) of Capture of Bacteria-Derived Membrane Vesicles by Capturing Agent]

Although Example 6 demonstrated that the capturing agent of the present invention is capable of capturing membrane vesicles in a culture solution using membrane vesicles of Bacteroides vulgatus, an evaluation was conducted on feces and bacteria-derived membrane vesicles purified from feces to confirm whether the present capturing method is capable of capturing bacteria-derived membrane vesicles more broadly. The evaluation was performed by comparison with ultracentrifugation, which is regarded as the golden standard for extracellular vesicle purification.

<Example 7>

(1. Preparation of pooled feces and purification of bacterial DNA in feces)

Feces from 20 healthy individuals were mixed to prepare pooled feces from healthy individuals. The purification of bacterial DNA in the feces was performed by processing 200 mg of the pooled feces with the QIAamp PowerFecal Pro DNA Kit (Qiagen) according to the method described in the attached manual.

(2. Purification of bacteria-derived membrane vesicles from feces by ultracentrifugation)

The pooled feces were suspended in PBS to make a 5% suspension, thereby obtaining a 5% fecal suspension. The suspension was centrifuged at 3,000×g for 30 minutes, and the obtained supernatant was treated with a 0.45 μm filter. Thereafter, after centrifuging at 150,000×g for 120 minutes using an ultracentrifuge (Beckman Coulter), the supernatant was discarded, an appropriate amount of PBS was added, and the suspension was centrifuged again at 150,000×g for 60 minutes. After removing the supernatant, CD1 buffer included in the QIAamp PowerFecal Pro DNA Kit (Qiagen) was added, processed according to the method described in the attached manual, and DNA was obtained.

(3. Purification of bacteria-derived membrane vesicles from feces by the capturing agent)

1 mL of the 5% fecal suspension was added to a Micro Bio-Spin chromatography column (7326204, from Bio-Rad) filled with 40 mg of the capturing agent and allowed to stand at room temperature for 30 minutes. Thereafter, the column was centrifuged at 1,000×g for 1 minute to remove the unreacted solution. Furthermore, 500 μL of physiological

saline was added and centrifuged at 1,000×g for 1 minute to remove it; this step was repeated three times. Then, CD1 buffer included in the QIAamp PowerFecal Pro DNA Kit (Qiagen) was added to the column and allowed to stand at room temperature for 10 minutes. Thereafter, the column was centrifuged at 1,000×g for 1 minute to obtain the eluate. The eluate was processed according to the method described in the attached manual of the kit to obtain DNA.

(4. Amplicon sequencing analysis of the bacterial DNA in the feces and in the bacteria-derived membrane vesicles)

Amplicon sequencing analysis (16S ribosomal DNA V3-V4 region) was performed on the DNA obtained by each method (feces, ultracentrifugation purification, capturing agent purification) by outsourcing the analysis to Bioengineering Lab. Co., Ltd.

<Results>

The results are shown in FIG. 6 and Table 1. FIG. 6 is a diagram illustrating the top 20 bacterial species profiles in the case of ultracentrifugation purification, obtained from the results of amplicon sequencing analysis of the DNA obtained from feces or DNA obtained from bacteria-derived membrane vesicles obtained by each purification method (ultracentrifugation purification, capturing agent purification). Table 1 shows all reads analyzed by amplicon sequencing and the number of those reads. As illustrated in FIG. 6 and Table 1, the bacterial species profile in feces and the profile of bacteria-derived membrane vesicles obtained by ultracentrifugation purification were greatly different. On the other hand, the profiles of bacteria-derived membrane vesicles obtained by ultracentrifugation purification and by the capturing agent purification were very similar, suggesting that purification by the capturing agent is capable of comprehensively capturing bacteria-derived membrane vesicles in feces in the same manner as ultracentrifugation purification.

[Table 1]

| Biological species | Ultracentrifugal purification | Capture agent purification | Feces |
|---|---|---|---|
| d_Bacteria;p_Bacteroidota;c_Bacteroidia;o_Bacteroidales;f_Prevotellaceae;g_Prevotella;_ | 6301 | 4151 | 749 |
| d_Bacteria;p_Firmicutes;c_Clostridia;o_Clostridia_UCG-014;f_Clostridia_UCG-014;g_Clostridia_UCG-014;_ | 6266 | 3390 | 64 |
| d_Bacteria;p_Bacteroidota;c_Bacteroidia;o_Bacteroidales;f_Rikenellaceae;g_Alistipes;_ | 5178 | 6285 | 521 |
| d_Bacteria;p_Bacteroidota;c_Bacteroidia;o_Bacteroidales;f_Bacteroidaceae;g_Bacteroides;_ | 2734 | 4695 | 4867 |
| d_Bacteria;p_Firmicutes;c_Clostridia;o_Oscillospirales;f_Ruminococcaceae;g_Faecalibacterium;_ | 2594 | 1481 | 2367 |
| d_Bacteria;p_Bacteroidota;c_Bacteroidia;o_Bacteroidales;f_Barnesiellaceae;g_Barnesiella;_ | 1456 | 1009 | 478 |
| d_Bacteria;p_Firmicutes;c_Clostridia;o_Oscillospirales;f_[Eubacterium]_coprostanoligenes_group;g_[Eubacterium]_coprostanoligenes_group;s_gut_metagenome | 1157 | 1082 | 122 |
| d_Bacteria;p_Firmicutes;c_Clostridia;o_Oscillospirales;f_Ruminococcaceae;_;_ | 1115 | 206 | 366 |
| d_Bacteria;p_Firmicutes;c_Bacilli;o_Erysipelotrichales;f_Erysipelotrichaceae;g_Turicibacter;s_uncultured_bacterium | 923 | 820 | 36 |
| d_Bacteria;p_Firmicutes;c_Clostridia;o_Oscillospirales;f_Ruminococcaceae;g_Subdoligranulum;_ | 790 | 337 | 795 |
| d_Bacteria;p_Firmicutes;c_Clostridia;o_Peptostreptococcales-Tissierellales;f_Peptostreptococcaceae;g_Romboutsia;_ | 786 | 1133 | 65 |
| d_Bacteria;p_Bacteroidota;c_Bacteroidia;o_Bacteroidales;f_Prevotellaceae;g_Prevotella;s_Prevotella_copri | 690 | 648 | 762 |
| d_Bacteria;p_Bacteroidota;c_Bacteroidia;o_Bacteroidales;f_Rikenellaceae;g_Alistipes;s_Alistipes_inops | 663 | 601 | 38 |
| d_Bacteria;p_Firmicutes;c_Clostridia;o_Oscillospirales;f_Oscillospiraceae;_;_ | 565 | 88 | 57 |
| d_Bacteria;p_Bacteroidota;c_Bacteroidia;o_Bacteroidales;f_Rikenellaceae;g_Alistipes;s_Alistipes_shahii | 548 | 583 | 92 |
| d_Bacteria;p_Firmicutes;c_Clostridia;o_Oscillospirales;f_Ruminococcaceae;g_Ruminococcus;_ | 507 | 81 | 190 |

| | | | |
|---|---|---|---|
| d_Bacteria;p_Firmicutes;c_Clostridia;o_Oscillospirales;f_Ruminococcaceae;g_Incertae_Sedis;s_[Clostridium]_leptum | 473 | 214 | 27 |
| d_Bacteria;p_Bacteroidota;c_Bacteroidia;o_Bacteroidales;f_Rikenellaceae;g_Alistipes;s_Alistipes_indistinctus | 403 | 157 | 32 |
| d_Bacteria;p_Bacteroidota;c_Bacteroidia;o_Bacteroidales;f_Bacteroidaceae;g_Bacteroides;s_Bacteroides_coprocola | 392 | 547 | 435 |
| d_Bacteria;p_Bacteroidota;c_Bacteroidia;o_Bacteroidales;f_Rikenellaceae;g_Alistipes;s_Alistipes_sp. | 374 | 229 | 23 |
| d_Bacteria;p_Firmicutes;c_Negativicutes;o_Acidaminococcales;f_Acidaminococcaceae;g_Phascolarctobacterium;_ | 360 | 90 | 301 |
| d_Bacteria;p_Firmicutes;c_Clostridia;o_Lachnospirales;f_Lachnospiraceae;_;_ | 352 | 241 | 1258 |
| d_Bacteria;p_Firmicutes;c_Clostridia;o_Oscillospirales;f_Ruminococcaceae;g_Ruminococcus;s_Ruminococcus_callidus | 321 | 41 | 169 |
| d_Bacteria;p_Actinobacteriota;c_Actinobacteria;o_Bifidobacteriales;f_Bifidobacteriaceae;g_Bifidobacterium;_ | 320 | 276 | 2011 |
| d_Bacteria;p_Firmicutes;c_Clostridia;o_Oscillospirales;f_Ruminococcaceae;g_CAG-352;_ | 305 | 184 | 28 |
| d_Bacteria;p_Actinobacteriota;c_Coriobacteriia;o_Coriobacteriales;f_Coriobacteriaceae;g_Collinsella;_ | 302 | 47 | 1217 |
| d_Bacteria;p_Firmicutes;c_Clostridia;o_Oscillospirales;f_UCG-010;g_UCG-010;_ | 294 | 119 | 18 |
| d_Bacteria;p_Firmicutes;c_Clostridia;o_Oscillospirales;f_[Eubacterium]_coprostanoligenes_group;g_[Eubacterium]_coprostanoligenes_group;s_human_gut | 267 | 139 | 165 |
| d_Bacteria;p_Bacteroidota;c_Bacteroidia;o_Bacteroidales;f_Rikenellaceae;g_Alistipes;s_Bacteroides_sp. | 263 | 288 | 35 |
| d_Bacteria;p_Actinobacteriota;c_Actinobacteria;o_Bifidobacteriales;f_Bifidobacteriaceae;g_Bifidobacterium;s_Bifidobacterium_longum | 261 | 306 | 405 |
| d_Bacteria;p_Bacteroidota;c_Bacteroidia;o_Bacteroidales;f_Tannerellaceae;g_Parabacteroides;_ | 249 | 331 | 485 |

| | | | |
|---|---|---|---|
| d_Bacteria;p_Firmicutes;c_Clostridia;o_Oscillospirales;f_[Eubacterium]_coprostanoligenes_group;g_[Eubacterium]_coprostanoligenes_group;_ | 234 | 483 | 79 |
| d_Bacteria;p_Bacteroidota;c_Bacteroidia;o_Bacteroidales;f_Prevotellaceae;g_Prevotellaceae_NK3B31_group;s_metagenome | 225 | 94 | 10 |
| d_Bacteria;p_Bacteroidota;c_Bacteroidia;o_Bacteroidales;f_Rikenellaceae;g_Alistipes;s_Alistipes_obesi | 208 | 396 | 37 |
| d_Bacteria;p_Verrucomicrobiota;c_Verrucomicrobiae;o_Verrucomicrobiales;f_Akkermansiaceae;g_Akkermansia;_ | 197 | 85 | 323 |
| d_Bacteria;p_Firmicutes;c_Clostridia;o_Oscillospirales;f_Ruminococcaceae;g_uncultured;s_human_gut | 176 | 4 | 24 |
| d_Bacteria;p_Bacteroidota;c_Bacteroidia;o_Bacteroidales;f_Prevotellaceae;g_Prevotellaceae_UCG-003;s_uncultured_bacterium | 173 | 485 | 152 |
| d_Bacteria;p_Firmicutes;c_Clostridia;o_Christensenellales;f_Christensenellaceae;g_Christensenellaceae_R-7_group;s_bacterium_YE57 | 172 | 5 | 8 |
| d_Bacteria;p_Firmicutes;c_Clostridia;o_Lachnospirales;f_Lachnospiraceae;g_[Eubacterium]_ventriosum_group;_ | 160 | 240 | 70 |
| d_Bacteria;p_Bacteroidota;c_Bacteroidia;o_Bacteroidales;f_Prevotellaceae;g_Prevotellaceae_NK3B31_group;s_uncultured_organism | 152 | 76 | 15 |
| d_Bacteria;p_Firmicutes;c_Clostridia;o_Oscillospirales;f_Oscillospiraceae;g_Oscillospira;_ | 146 | 5 | 10 |
| d_Bacteria;p_Firmicutes;c_Clostridia;o_Oscillospirales;f_Oscillospiraceae;g_Oscillibacter;_ | 141 | 85 | 79 |
| d_Bacteria;p_Firmicutes;c_Clostridia;o_Oscillospirales;f_Oscillospiraceae;g_Oscillibacter;s_uncultured_organism | 139 | 6 | 13 |
| d_Bacteria;p_Firmicutes;c_Clostridia;o_Lachnospirales;f_Lachnospiraceae;g_Roseburia;_ | 128 | 248 | 586 |
| d_Bacteria;p_Firmicutes;c_Clostridia;o_Oscillospirales;f_Ruminococcaceae;g_Incertae_Sedis;_ | 128 | 21 | 171 |
| d_Bacteria;p_Firmicutes;c_Clostridia;o_Oscillospirales;f_Ruminococcaceae;g_DTU089;_ | 128 | 0 | 0 |

| | | | |
|---|---|---|---|
| d_Bacteria;p_Firmicutes;c_Clostridia;o_Monoglobales;f_Monoglobaceae;g_Monoglobus;_ | 127 | 105 | 157 |
| d_Bacteria;p_Firmicutes;c_Clostridia;o_Peptostreptococcales-Tissierellales;f_Peptostreptococcaceae;g_Intestinibacter;_ | 125 | 185 | 45 |
| d_Bacteria;p_Bacteroidota;c_Bacteroidia;o_Bacteroidales;f_Marinifilaceae;g_Butyricimonas;_ | 119 | 250 | 92 |
| d_Bacteria;p_Firmicutes;c_Clostridia;o_Oscillospirales;f_Oscillospiraceae;g_Colidextribacter;_ | 118 | 31 | 31 |
| d_Bacteria;p_Firmicutes;c_Clostridia;o_Oscillospirales;f_Oscillospiraceae;g_UCG-002;_ | 111 | 24 | 66 |
| d_Bacteria;p_Firmicutes;c_Clostridia;o_Oscillospirales;f_Oscillospiraceae;g_Flavonifractor;s_uncultured_bacterium | 109 | 0 | 18 |
| d_Bacteria;p_Bacteroidota;c_Bacteroidia;o_Bacteroidales;f_Rikenellaceae;g_Alistipes;s_Alistipes_finegoldii | 103 | 89 | 25 |
| d_Bacteria;p_Firmicutes;c_Clostridia;o_Lachnospirales;f_Lachnospiraceae;g_Agathobacter;_ | 99 | 193 | 621 |
| d_Bacteria;p_Firmicutes;c_Clostridia;o_Oscillospirales;f_Ruminococcaceae;g_uncultured;_ | 97 | 61 | 7 |
| d_Bacteria;p_Firmicutes;c_Negativicutes;o_Veillonellales-Selenomonadales;f_Selenomonadaceae;g_Megamonas;s_Megamonas_funiformis | 89 | 84 | 356 |
| d_Bacteria;p_Firmicutes;c_Clostridia;o_Oscillospirales;f_Oscillospiraceae;g_NK4A214_group;_ | 89 | 47 | 97 |
| d_Bacteria;p_Firmicutes;c_Negativicutes;o_Veillonellales-Selenomonadales;f_Veillonellaceae;g_Dialister;_ | 88 | 101 | 97 |
| d_Bacteria;p_Firmicutes;c_Clostridia;o_Peptostreptococcales-Tissierellales;f_Anaerovoracaceae;g_Family_XIII_AD3011_group;_ | 86 | 53 | 0 |
| d_Bacteria;p_Firmicutes;c_Clostridia;o_Oscillospirales;f_Ruminococcaceae;g_uncultured;s_uncultured_bacterium | 84 | 0 | 6 |
| d_Bacteria;p_Bacteroidota;c_Bacteroidia;o_Bacteroidales;f_Muribaculaceae;g_Muribaculaceae;s_uncultured_bacterium | 83 | 236 | 83 |
| d_Bacteria;p_Firmicutes;c_Clostridia;o_Lachnospirales;f_Lachnospiraceae;g_[Eubacterium]_ruminantium_group;s_uncultured_bacterium | 82 | 108 | 191 |

| | | | |
|---|---|---|---|
| d__Bacteria;p__Firmicutes;c__Clostridia;o__Christensenellales;f__Christensenellaceae;g__Christensenellaceae_R-7_group;__ | 82 | 100 | 30 |
| d__Bacteria;p__Bacteroidota;c__Bacteroidia;o__Bacteroidales;f__Bacteroidaceae;g__Bacteroides;s__Bacteroides_uniformis | 75 | 112 | 182 |
| d__Bacteria;p__Bacteroidota;c__Bacteroidia;o__Bacteroidales;f__Marinifilaceae;g__Odoribacter;__ | 73 | 123 | 32 |
| d__Bacteria;p__Firmicutes;c__Clostridia;o__Peptostreptococcales-Tissierellales;f__Peptostreptococcaceae;g__Terrisporobacter;s__uncultured_bacterium | 72 | 71 | 18 |
| d__Bacteria;p__Firmicutes;c__Clostridia;o__Oscillospirales;f__Oscillospiraceae;g__NK4A214_group;s__uncultured_organism | 72 | 0 | 0 |
| d__Bacteria;p__Firmicutes;c__Clostridia;o__Oscillospirales;f__Oscillospiraceae;g__UCG-005;__ | 65 | 0 | 21 |
| d__Bacteria;p__Bacteroidota;c__Bacteroidia;o__Bacteroidales;f__Bacteroidaceae;g__Bacteroides;s__Bacteroides_vulgatus | 64 | 254 | 355 |
| d__Bacteria;p__Firmicutes;c__Clostridia;o__Oscillospirales;f__Ruminococcaceae;g__[Eubacterium]_siraeum_group;s__[Eubacterium]_siraeum | 61 | 136 | 7 |
| d__Bacteria;p__Bacteroidota;c__Bacteroidia;o__Bacteroidales;f__Prevotellaceae;g__Paraprevotella;__ | 61 | 57 | 83 |
| d__Bacteria;p__Firmicutes;c__Clostridia;__;__;__;__ | 60 | 0 | 5 |
| d__Bacteria;p__Firmicutes;c__Bacilli;o__RF39;f__RF39;g__RF39;s__human_gut | 59 | 73 | 0 |
| d__Bacteria;p__Firmicutes;c__Clostridia;o__Peptostreptococcales-Tissierellales;f__Anaerovoracaceae;g__Family_XIII_AD3011_group;s__uncultured_bacterium | 58 | 29 | 0 |
| d__Bacteria;p__Firmicutes;c__Clostridia;o__Oscillospirales;f__Ruminococcaceae;g__Subdoligranulum;s__uncultured_bacterium | 57 | 97 | 245 |
| d__Bacteria;p__Bacteroidota;c__Bacteroidia;o__Bacteroidales;f__Bacteroidaceae;g__Bacteroides;s__Bacteroides_plebeius | 57 | 262 | 264 |
| d__Bacteria;p__Bacteroidota;c__Bacteroidia;o__Bacteroidales;f__Prevotellaceae;g__Paraprevotella;s__Paraprevotella_xylaniphila | 56 | 122 | 94 |

| | | | |
|---|---|---|---|
| d_Bacteria;p_Firmicutes;c_Clostridia;o_Christensenellales;f_Christensenellaceae;g_uncultured;s_uncultured_bacterium | 56 | 0 | 6 |
| d_Bacteria;p_Firmicutes;c_Clostridia;o_Clostridia_vadinBB60_group;f_Clostridia_vadinBB60_group;g_Clostridia_vadinBB60_group;s_uncultured_Thermoanaerobacterales | 55 | 10 | 7 |
| d_Bacteria;p_Firmicutes;c_Clostridia;o_Christensenellales;f_Christensenellaceae;g_Christensenellaceae_R-7_group;s_bacterium_enrichment | 51 | 0 | 0 |
| d_Bacteria;p_Bacteroidota;c_Bacteroidia;o_Bacteroidales;f_Bacteroidaceae;g_Bacteroides;s_Bacteroides_dorei | 50 | 423 | 424 |
| d_Bacteria;p_Firmicutes;c_Clostridia;o_Peptostreptococcales-Tissierellales;f_Anaerovoracaceae;_;_ | 50 | 6 | 0 |
| d_Bacteria;p_Firmicutes;c_Clostridia;o_Oscillospirales;f_Oscillospiraceae;g_uncultured;_ | 50 | 9 | 52 |
| d_Bacteria;p_Actinobacteriota;c_Coriobacteriia;o_Coriobacteriales;f_Eggerthellaceae;g_Eggerthella;s_uncultured_bacterium | 49 | 0 | 0 |
| d_Bacteria;p_Firmicutes;c_Clostridia;o_Oscillospirales;f_Oscillospiraceae;g_Flavonifractor;_ | 48 | 0 | 94 |
| d_Bacteria;p_Bacteroidota;c_Bacteroidia;o_Bacteroidales;f_Bacteroidaceae;g_Bacteroides;s_Bacteroides_eggerthii | 45 | 221 | 135 |
| d_Bacteria;p_Firmicutes;c_Clostridia;o_Lachnospirales;f_Lachnospiraceae;g_Lachnospiraceae_NK4A136_group;_ | 44 | 138 | 78 |
| d_Bacteria;p_Firmicutes;c_Clostridia;o_Oscillospirales;f_Ruminococcaceae;g_Ruminococcus;s_Ruminococcus_bicirculans | 43 | 82 | 128 |
| d_Bacteria;p_Bacteroidota;c_Bacteroidia;o_Bacteroidales;f_Bacteroidaceae;g_Bacteroides;s_Bacteroides_stercoris | 42 | 473 | 540 |
| d_Bacteria;p_Firmicutes;c_Bacilli;o_RF39;f_RF39;g_RF39;s_uncultured_bacterium | 42 | 69 | 0 |
| d_Bacteria;p_Firmicutes;c_Clostridia;o_Christensenellales;f_Christensenellaceae;g_Christensenellaceae_R-7_group;s_uncultured_marine | 42 | 18 | 2 |

| | | | |
|---|---|---|---|
| d_Bacteria;p_Actinobacteriota;c_Coriobacteriia;o_Coriobacteriales;f_Coriobacteriales_Incertae_Sedis;g_uncultured;s_gut_metagenome | 38 | 0 | 7 |
| d_Bacteria;p_Firmicutes;c_Clostridia;o_Lachnospirales;f_Lachnospiraceae;g_[Ruminococcus]_torques_group;_ | 35 | 104 | 415 |
| d_Bacteria;p_Firmicutes;c_Clostridia;o_Lachnospirales;f_Lachnospiraceae;g_[Eubacterium]_xylanophilum_group;_ | 35 | 346 | 46 |
| d_Bacteria;p_Firmicutes;c_Negativicutes;o_Veillonellales-Selenomonadales;f_Selenomonadaceae;g_Megamonas;_ | 34 | 207 | 1930 |
| d_Bacteria;p_Firmicutes;c_Clostridia;o_Oscillospirales;f_Ruminococcaceae;g_CAG-352;s_uncultured_bacterium | 34 | 17 | 16 |
| d_Bacteria;p_Firmicutes;c_Negativicutes;o_Veillonellales-Selenomonadales;f_Veillonellaceae;g_Veillonella;_ | 32 | 27 | 24 |
| d_Bacteria;p_Firmicutes;c_Clostridia;o_Oscillospirales;f_UCG-010;g_UCG-010;s_unidentified | 32 | 0 | 0 |
| d_Bacteria;p_Firmicutes;c_Clostridia;o_Oscillospirales;f_Ruminococcaceae;g_Anaerotruncus;_ | 32 | 0 | 0 |
| d_Bacteria;p_Firmicutes;c_Clostridia;o_Oscillospirales;f_Ruminococcaceae;g_Incertae_Sedis;s_uncultured_organism | 29 | 45 | 11 |
| d_Bacteria;p_Proteobacteria;c_Gammaproteobacteria;o_Burkholderiales;f_Sutterellaceae;g_Parasutterella;_ | 29 | 0 | 45 |
| d_Bacteria;p_Firmicutes;c_Clostridia;o_Oscillospirales;_;_;_ | 29 | 0 | 0 |
| d_Bacteria;p_Firmicutes;c_Clostridia;o_Oscillospirales;f_UCG-010;g_UCG-010;s_uncultured_organism | 29 | 17 | 0 |
| d_Bacteria;p_Proteobacteria;c_Gammaproteobacteria;o_Pasteurellales;f_Pasteurellaceae;g_Haemophilus;_ | 28 | 0 | 76 |
| d_Bacteria;p_Firmicutes;c_Clostridia;o_Lachnospirales;f_Lachnospiraceae;g_[Ruminococcus]_torques_group;s_Ruminococcus_sp. | 28 | 0 | 0 |
| d_Bacteria;p_Actinobacteriota;c_Coriobacteriia;o_Coriobacteriales;f_Coriobacteriales_Incertae_Sedis;g_Raoultibacter;_ | 28 | 0 | 0 |
| d_Bacteria;p_Actinobacteriota;c_Coriobacteriia;o_Coriobacteriales;f_Atopobiaceae;g_Libanicoccus;s_uncultured_bacterium | 26 | 0 | 45 |

| | | | |
|---|---|---|---|
| d_Bacteria;p_Firmicutes;c_Clostridia;o_Oscillospirales;f_Oscillospiraceae;g_Colidextribacter;s_uncultured_Clostridia | 26 | 0 | 6 |
| d_Bacteria;p_Firmicutes;c_Clostridia;o_Monoglobales;f_Monoglobaceae;g_Monoglobus;s_uncultured_bacterium | 26 | 0 | 0 |
| d_Bacteria;p_Bacteroidota;c_Bacteroidia;o_Bacteroidales;f_Tannerellaceae;g_Parabacteroides;s_Parabacteroides_merdae | 25 | 143 | 689 |
| d_Bacteria;p_Firmicutes;c_Clostridia;o_Clostridiales;f_Clostridiaceae;g_Clostridium_sensu_stricto_1;_ | 25 | 656 | 28 |
| d_Bacteria;p_Firmicutes;c_Clostridia;o_Oscillospirales;f_Ruminococcaceae;g_uncultured;s_uncultured_organism | 25 | 0 | 7 |
| d_Bacteria;p_Firmicutes;c_Clostridia;o_Oscillospirales;f_Oscillospiraceae;g_Oscillospira;s_uncultured_bacterium | 25 | 0 | 0 |
| d_Bacteria;p_Firmicutes;c_Clostridia;o_Oscillospirales;f_Ruminococcaceae;g_Incertae_Sedis;s_uncultured_bacterium | 25 | 0 | 0 |
| d_Bacteria;p_Firmicutes;c_Clostridia;o_Oscillospirales;f_Oscillospiraceae;g_UCG-002;s_uncultured_organism | 24 | 132 | 43 |
| d_Bacteria;p_Firmicutes;c_Bacilli;o_Lactobacillales;f_Enterococcaceae;g_Enterococcus;_ | 23 | 0 | 8 |
| d_Bacteria;p_Firmicutes;c_Clostridia;o_Oscillospirales;f_Oscillospiraceae;g_Oscillibacter;s_human_gut | 23 | 91 | 14 |
| d_Bacteria;p_Bacteroidota;c_Bacteroidia;o_Bacteroidales;f_Barnesiellaceae;g_Coprobacter;_ | 23 | 63 | 37 |
| d_Bacteria;p_Proteobacteria;c_Gammaproteobacteria;o_Burkholderiales;f_Sutterellaceae;g_Parasutterella;s_Parasutterella_secunda | 23 | 2 | 6 |
| d_Bacteria;p_Firmicutes;c_Clostridia;o_Oscillospirales;f_Ruminococcaceae;g_Negativibacillus;s_uncultured_bacterium | 22 | 21 | 75 |
| d_Bacteria;p_Bacteroidota;c_Bacteroidia;o_Bacteroidales;f_Rikenellaceae;g_Alistipes;s_Alistipes_timonensis | 21 | 0 | 0 |
| d_Bacteria;p_Firmicutes;c_Clostridia;o_Oscillospirales;f_Ruminococcaceae;g_Fournierella;s_uncultured_organism | 21 | 0 | 0 |

| | | | |
|---|---|---|---|
| d_Bacteria;p_Bacteroidota;c_Bacteroidia;o_Bacteroidales;f_Barnesiellaceae;g_Coprobacter;s_Coprobacter_secundus | 20 | 95 | 6 |
| d_Bacteria;p_Firmicutes;c_Clostridia;o_Lachnospirales;f_Lachnospiraceae;g_Coprococcus;_ | 18 | 82 | 253 |
| d_Bacteria;p_Firmicutes;c_Clostridia;o_Oscillospirales;f_Butyricicoccaceae;g_Butyricicoccus;_ | 17 | 25 | 159 |
| d_Bacteria;p_Bacteroidota;c_Bacteroidia;o_Flavobacteriales;f_Flavobacteriaceae;g_uncultured;s_gut_metagenome | 17 | 20 | 0 |
| d_Bacteria;p_Bacteroidota;c_Bacteroidia;o_Bacteroidales;f_Bacteroidaceae;g_Bacteroides;s_Bacteroides_finegoldii | 16 | 64 | 44 |
| d_Bacteria;p_Firmicutes;c_Clostridia;o_Lachnospirales;f_Lachnospiraceae;g_Lachnospiraceae_ND3007_group;_ | 15 | 26 | 143 |
| d_Bacteria;p_Bacteroidota;c_Bacteroidia;o_Bacteroidales;f_Prevotellaceae;g_Paraprevotella;s_uncultured_bacterium | 14 | 62 | 64 |
| d_Bacteria;p_Firmicutes;c_Clostridia;o_Oscillospirales;f_Oscillospiraceae;g_Intestinimonas;s_Intestinimonas_butyriciproducens | 14 | 29 | 11 |
| d_Bacteria;p_Firmicutes;c_Clostridia;o_Lachnospirales;f_Lachnospiraceae;g_Blautia;_ | 13 | 227 | 2825 |
| d_Bacteria;p_Desulfobacterota;c_Desulfovibrionia;o_Desulfovibrionales;f_Desulfovibrionaceae;g_Bilophila;_ | 13 | 2 | 288 |
| d_Bacteria;p_Firmicutes;c_Clostridia;o_Lachnospirales;f_Lachnospiraceae;g_[Eubacterium]_eligens_group;_ | 13 | 287 | 48 |
| d_Bacteria;p_Firmicutes;c_Clostridia;o_Lachnospirales;f_Lachnospiraceae;g_Dorea;_ | 12 | 14 | 361 |
| d_Bacteria;p_Firmicutes;c_Clostridia;o_Oscillospirales;f_Ruminococcaceae;g_Anaerotruncus;s_Anaerotruncus_sp. | 11 | 34 | 0 |
| d_Bacteria;p_Firmicutes;c_Clostridia;o_Oscillospirales;f_Ruminococcaceae;g_UBA1819;s_uncultured_organism | 10 | 36 | 15 |
| d_Bacteria;p_Firmicutes;c_Negativicutes;o_Veillonellales-Selenomonadales;f_Veillonellaceae;g_Dialister;s_uncultured_bacterium | 10 | 40 | 65 |
| d_Bacteria;p_Firmicutes;c_Clostridia;o_Oscillospirales;f_Oscillospiraceae;g_UCG-003;_ | 10 | 45 | 2 |

【表１＃１０】

| | | | |
|---|---|---|---|
| d_Bacteria;p_Firmicutes;c_Bacilli;o_Erysipelotrichales;f_Erysipelatoclostridiaceae;g_Erysipelatoclostridium;_ | 9 | 13 | 81 |
| d_Bacteria;p_Firmicutes;c_Clostridia;o_Lachnospirales;f_Lachnospiraceae;g_Dorea;s_Dorea_formicigenerans | 8 | 0 | 101 |
| d_Bacteria;p_Proteobacteria;c_Gammaproteobacteria;o_Burkholderiales;f_Sutterellaceae;g_Sutterella;s_uncultured_bacterium | 7 | 64 | 155 |
| d_Bacteria;p_Bacteroidota;c_Bacteroidia;o_Bacteroidales;f_Bacteroidaceae;g_Bacteroides;s_Bacteroides_massiliensis | 7 | 73 | 95 |
| d_Bacteria;p_Proteobacteria;c_Gammaproteobacteria;o_Enterobacterales;f_Enterobacteriaceae;g_Escherichia-Shigella;_ | 6 | 0 | 249 |
| d_Bacteria;p_Bacteroidota;c_Bacteroidia;o_Bacteroidales;f_Prevotellaceae;g_Prevotella;s_Prevotella_stercorea | 6 | 62 | 151 |
| d_Bacteria;p_Patescibacteria;c_Saccharimonadia;o_Saccharimonadales;f_Saccharimonadaceae;g_Saccharimonadaceae;s_uncultured_bacterium | 5 | 20 | 0 |
| d_Bacteria;p_Bacteroidota;c_Bacteroidia;o_Bacteroidales;f_Bacteroidaceae;g_Bacteroides;s_Bacteroides_cellulosilyticus | 5 | 38 | 84 |
| d_Bacteria;p_Proteobacteria;c_Gammaproteobacteria;o_Burkholderiales;f_Sutterellaceae;g_Sutterella;s_Sutterella_wadsworthensis | 5 | 60 | 55 |
| d_Bacteria;p_Fusobacteriota;c_Fusobacteriia;o_Fusobacteriales;f_Fusobacteriaceae;g_Fusobacterium;s_Fusobacterium_mortiferum | 5 | 45 | 25 |
| d_Bacteria;p_Firmicutes;c_Negativicutes;o_Veillonellales-Selenomonadales;f_Veillonellaceae;g_Megasphaera;s_Megasphaera_micronuciformis | 4 | 59 | 47 |
| d_Bacteria;p_Actinobacteriota;c_Coriobacteriia;o_Coriobacteriales;f_Eggerthellaceae;g_Slackia;s_Slackia_isoflavoniconvertens | 4 | 0 | 76 |
| d_Bacteria;p_Firmicutes;c_Clostridia;o_Lachnospirales;f_Lachnospiraceae;g_Sellimonas;_ | 4 | 0 | 20 |
| d_Bacteria;p_Firmicutes;c_Clostridia;o_Lachnospirales;f_Lachnospiraceae;g_Fusicatenibacter;_ | 3 | 52 | 650 |

| | | | |
|---|---|---|---|
| d__Bacteria;p__Actinobacteriota;c__Actinobacteria;o__Frankiales;f__Geodermatophilaceae;g__Blastococcus;__ | 2 | 30 | 0 |
| d__Bacteria;p__Firmicutes;c__Clostridia;o__Lachnospirales;f__Lachnospiraceae;g__[Ruminococcus]_gnavus_group;__ | 0 | 145 | 284 |
| d__Bacteria;p__Firmicutes;c__Bacilli;o__Lactobacillales;f__Streptococcaceae;g__Streptococcus;s__Streptococcus_salivarius | 0 | 27 | 362 |
| d__Bacteria;p__Firmicutes;c__Clostridia;o__Lachnospirales;f__Lachnospiraceae;g__Anaerostipes;__ | 0 | 50 | 764 |
| d__Bacteria;p__Firmicutes;c__Clostridia;o__Lachnospirales;f__Lachnospiraceae;g__[Eubacterium]_hallii_group;__ | 0 | 46 | 728 |
| d__Bacteria;p__Bacteroidota;c__Bacteroidia;o__Bacteroidales;f__Bacteroidaceae;g__Bacteroides;s__Bacteroides_fragilis | 0 | 13 | 61 |
| d__Bacteria;p__Firmicutes;c__Clostridia;o__Lachnospirales;f__Lachnospiraceae;g__Sellimonas;s__uncultured_bacterium | 0 | 10 | 75 |
| d__Bacteria;p__Firmicutes;c__Bacilli;o__Erysipelotrichales;f__Erysipelatoclostridiaceae;g__Erysipelotrichaceae_UCG-003;__ | 0 | 73 | 142 |
| d__Bacteria;p__Firmicutes;c__Negativicutes;o__Acidaminococcales;f__Acidaminococcaceae;g__Phascolarctobacterium;s__uncultured_Firmicutes | 0 | 0 | 48 |
| d__Bacteria;p__Firmicutes;c__Clostridia;o__Lachnospirales;f__Lachnospiraceae;g__Lachnoclostridium;__ | 0 | 112 | 560 |
| d__Bacteria;p__Firmicutes;c__Clostridia;o__Lachnospirales;f__Lachnospiraceae;g__Lachnospira;__ | 0 | 309 | 131 |
| d__Bacteria;p__Firmicutes;c__Bacilli;o__Erysipelotrichales;f__Erysipelotrichaceae;g__Holdemanella;__ | 0 | 24 | 161 |
| d__Bacteria;p__Firmicutes;c__Clostridia;o__Lachnospirales;f__Lachnospiraceae;g__[Ruminococcus]_gauvreauii_group;__ | 0 | 0 | 120 |
| d__Bacteria;p__Actinobacteriota;c__Coriobacteriia;o__Coriobacteriales;f__Eggerthellaceae;g__Eggerthella;__ | 0 | 0 | 53 |
| d__Bacteria;p__Firmicutes;c__Clostridia;o__Lachnospirales;f__Lachnospiraceae;g__Lachnospira;s__uncultured_bacterium | 0 | 188 | 70 |

| | | | |
|---|---|---|---|
| d_Bacteria;p_Firmicutes;c_Bacilli;o_Erysipelotrichales;f_Erysipelotrichaceae;g_[Clostridium]_innocuum_group;_ _ | 0 | 0 | 26 |
| d_Bacteria;p_Firmicutes;c_Negativicutes;o_Veillonellales-Selenomonadales;f_Veillonellaceae;g_Megasphaera;_ | 0 | 0 | 358 |
| d_Bacteria;p_Firmicutes;c_Clostridia;o_Lachnospirales;f_Lachnospiraceae;g_CAG-56;s_uncultured_bacterium | 0 | 23 | 71 |
| d_Bacteria;p_Firmicutes;c_Bacilli;o_Erysipelotrichales;f_Erysipelotrichaceae;g_Holdemanella;s_uncultured_bacterium | 0 | 29 | 44 |
| d_Bacteria;p_Firmicutes;c_Negativicutes;o_Veillonellales-Selenomonadales;f_Veillonellaceae;g_Megasphaera;s_Megasphaera_elsdenii | 0 | 8 | 139 |
| d_Bacteria;p_Firmicutes;c_Negativicutes;o_Veillonellales-Selenomonadales;f_Veillonellaceae;g_Megasphaera;s_uncultured_organism | 0 | 0 | 117 |
| d_Bacteria;p_Firmicutes;c_Bacilli;o_Lactobacillales;f_Streptococcaceae;g_Streptococcus;_ | 0 | 10 | 137 |
| d_Bacteria;p_Firmicutes;c_Clostridia;o_Lachnospirales;f_Lachnospiraceae;g_Lachnospiraceae_UCG-010;s_uncultured_organism | 0 | 134 | 53 |
| d_Bacteria;p_Firmicutes;c_Negativicutes;o_Acidaminococcales;f_Acidaminococcaceae;g_Acidaminococcus;_ | 0 | 52 | 88 |
| d_Bacteria;p_Desulfobacterota;c_Desulfovibrionia;o_Desulfovibrionales;f_Desulfovibrionaceae;g_Desulfovibrio;s_bacterium | 0 | 0 | 133 |
| d_Bacteria;p_Firmicutes;c_Clostridia;o_Christensenellales;f_Christensenellaceae;g_Christensenellaceae_R-7_group;s_uncultured_prokaryote | 0 | 20 | 15 |
| d_Bacteria;p_Firmicutes;c_Bacilli;o_Bacillales;f_Bacillaceae;g_Bacillus;_ | 0 | 0 | 56 |
| d_Bacteria;p_Firmicutes;c_Clostridia;o_Lachnospirales;f_Lachnospiraceae;g_Lachnospiraceae_NK4A136_group;s_uncultured_organism | 0 | 54 | 65 |
| d_Bacteria;p_Proteobacteria;c_Gammaproteobacteria;o_Enterobacterales;f_Enterobacteriaceae;_;_ | 0 | 0 | 95 |
| d_Bacteria;p_Bacteroidota;c_Bacteroidia;o_Bacteroidales;f_Prevotellaceae;g_Alloprevotella;s_Prevotellaceae_bacterium | 0 | 58 | 34 |

| | | | |
|---|---|---|---|
| d_Bacteria;p_Firmicutes;c_Clostridia;o_Clostridiales;f_Clostridiaceae;g_Clostridium_sensu_stricto_1;s_Clostridium_butyricum | 0 | 93 | 0 |
| d_Bacteria;p_Firmicutes;c_Clostridia;o_Lachnospirales;f_Lachnospiraceae;g_Roseburia;s_gut_metagenome | 0 | 0 | 39 |
| d_Bacteria;p_Actinobacteriota;c_Coriobacteriia;o_Coriobacteriales;f_Eggerthellaceae;g_Senegalimassilia;s_uncultured_bacterium | 0 | 0 | 46 |
| d_Bacteria;p_Firmicutes;c_Clostridia;o_Lachnospirales;f_Lachnospiraceae;g_Lachnoclostridium;s_Eubacterium_sp. | 0 | 31 | 26 |
| d_Bacteria;p_Firmicutes;c_Negativicutes;o_Veillonellales-Selenomonadales;f_Selenomonadaceae;g_Mitsuokella;s_uncultured_bacterium | 0 | 0 | 76 |
| d_Bacteria;p_Firmicutes;c_Clostridia;o_Lachnospirales;f_Lachnospiraceae;g_uncultured;s_uncultured_Eubacterium | 0 | 0 | 24 |
| d_Bacteria;p_Firmicutes;c_Clostridia;o_Lachnospirales;f_Lachnospiraceae;g_Marvinbryantia;_ | 0 | 0 | 28 |
| d_Bacteria;p_Firmicutes;c_Clostridia;o_Lachnospirales;f_Lachnospiraceae;g_Frisingicoccus;_ | 0 | 57 | 22 |
| d_Bacteria;p_Bacteroidota;c_Bacteroidia;o_Bacteroidales;f_Tannerellaceae;g_Parabacteroides;s_Parabacteroides_goldsteinii | 0 | 44 | 12 |
| d_Bacteria;p_Firmicutes;c_Clostridia;o_Peptostreptococcales-Tissierellales;f_Anaerovoracaceae;g_Family_XIII_AD3011_group;s_gut_metagenome | 0 | 33 | 0 |
| d_Bacteria;p_Bacteroidota;c_Bacteroidia;o_Bacteroidales;f_Bacteroidaceae;g_Bacteroides;s_Bacteroides_faecis | 0 | 0 | 27 |
| d_Bacteria;p_Firmicutes;c_Negativicutes;o_Veillonellales-Selenomonadales;f_Veillonellaceae;g_Megasphaera;s_Megasphaera_stantonii | 0 | 10 | 44 |
| d_Bacteria;p_Firmicutes;c_Bacilli;o_Erysipelotrichales;f_Erysipelatoclostridiaceae;g_Erysipelotrichaceae_UCG-003;s_uncultured_bacterium | 0 | 0 | 54 |
| d_Bacteria;p_Firmicutes;c_Clostridia;o_Oscillospirales;f_Oscillospiraceae;g_uncultured;s_Clostridium_sp. | 0 | 0 | 33 |

| | | | |
|---|---|---|---|
| d_Bacteria;p_Actinobacteriota;c_Coriobacteriia;o_Coriobacteriales;f_Coriobacteriales_Incertae_Sedis;g_uncultured;s_uncultured_organism | 0 | 5 | 26 |
| d_Bacteria;p_Firmicutes;c_Clostridia;o_Peptococcales;f_Peptococcaceae;g_Peptococcus;s_uncultured_organism | 0 | 49 | 8 |
| d_Bacteria;p_Proteobacteria;c_Gammaproteobacteria;o_Burkholderiales;f_Oxalobacteraceae;g_Massilia;_ | 0 | 48 | 0 |
| d_Bacteria;p_Firmicutes;c_Bacilli;o_Lactobacillales;f_Lactobacillaceae;g_Lactobacillus;s_Lactobacillus_ruminis | 0 | 0 | 45 |
| d_Bacteria;p_Firmicutes;c_Clostridia;o_Lachnospirales;f_Lachnospiraceae;g_Tuzzerella;_ | 0 | 40 | 0 |
| d_Bacteria;p_Firmicutes;c_Clostridia;o_Oscillospirales;f_Butyricicoccaceae;g_UCG-009;_ | 0 | 24 | 10 |
| d_Bacteria;p_Firmicutes;c_Clostridia;o_Oscillospirales;f_Oscillospiraceae;g_UCG-002;s_uncultured_rumen | 0 | 33 | 0 |
| d_Bacteria;p_Bacteroidota;c_Bacteroidia;o_Bacteroidales;f_Bacteroidaceae;g_Bacteroides;s_Bacteroides_nordii | 0 | 33 | 0 |
| d_Bacteria;p_Bacteroidota;c_Bacteroidia;o_Bacteroidales;f_Tannerellaceae;g_Parabacteroides;s_Parabacteroides_gordonii | 0 | 0 | 33 |
| d_Bacteria;p_Actinobacteriota;c_Coriobacteriia;o_Coriobacteriales;f_Eggerthellaceae;g_Adlercreutzia;s_uncultured_bacterium | 0 | 0 | 33 |
| d_Bacteria;p_Firmicutes;c_Bacilli;o_Erysipelotrichales;f_Erysipelotrichaceae;g_uncultured;s_Clostridiales_bacterium | 0 | 25 | 7 |
| d_Bacteria;p_Bacteroidota;c_Bacteroidia;o_Bacteroidales;f_Marinifilaceae;g_Butyricimonas;s_uncultured_bacterium | 0 | 25 | 6 |
| d_Bacteria;p_Firmicutes;c_Clostridia;o_Oscillospirales;f_Oscillospiraceae;g_V9D2013_group;s_uncultured_bacterium | 0 | 28 | 0 |
| d_Bacteria;p_Bacteroidota;c_Bacteroidia;o_Bacteroidales;f_Marinifilaceae;g_Odoribacter;s_Odoribacter_splanchnicus | 0 | 28 | 0 |

| | | | |
|---|---|---|---|
| d_Bacteria;p_Proteobacteria;c_Gammaproteobacteria;o_Aeromonadales;f_Succinivibrionaceae;g_Succinivibrio;_ | 0 | 0 | 26 |
| d_Bacteria;p_Proteobacteria;c_Gammaproteobacteria;o_Aeromonadales;f_Succinivibrionaceae;g_Anaerobiospirillum;s_uncultured_bacterium | 0 | 25 | 0 |
| d_Bacteria;p_Firmicutes;c_Clostridia;o_Lachnospirales;f_Lachnospiraceae;g_GCA-900066575;_ | 0 | 22 | 0 |
| d_Bacteria;p_Firmicutes;c_Clostridia;o_Oscillospirales;f_Ruminococcaceae;g_Phocea;_ | 0 | 22 | 0 |
| d_Bacteria;p_Firmicutes;c_Clostridia;o_Lachnospirales;f_Lachnospiraceae;g_[Eubacterium]_hallii_group;s_uncultured_bacterium | 0 | 0 | 22 |
| d_Bacteria;p_Desulfobacterota;c_Desulfovibrionia;o_Desulfovibrionales;f_Desulfovibrionaceae;g_Desulfovibrio;_ | 0 | 0 | 20 |

## Claims

**1.** A method for purifying extracellular vesicles and/or membrane vesicles in feces, the method comprising:

a step of filtering a sample of the feces with a filter; and
a step of bringing the filtered sample into contact with a capturing agent, wherein
the capturing agent contains a crosslinked polymer including monomer units having an acidic group and/or monomer units having a neutralizing base of the acidic group, and satisfies the following (i) and/or (ii):

(i) the crosslinked polymer includes at least monomer units having a neutralizing base between the acidic group and the first cationic surfactant; and
(ii) the capturing agent further contains a second cationic surfactant (provided that the second cationic surfactant is excluded when the second cationic surfactant is a crosslinked polymer including monomer units having a neutralizing base of the acidic group).

**2.** The method according to claim 1, wherein

the monomer units having the acidic group are unsaturated monocarboxylic acids having 3 to 10 carbon atoms, and
the monomer units having the neutralizing base of the acidic group are unsaturated monocarboxylate salts having 3 to 10 carbon atoms.

**3.** The method according to claim 1, wherein

the monomer units having the acidic group are acrylic acid, and
the monomer units having the neutralizing base of the acidic group are acrylates.

**4.** The method according to claim 1, wherein a cationic group in the cationic surfactant is $-N^{+}R_3$ (wherein each R independently represents a hydrogen atom or a hydrocarbon group having 1 to 6 carbon atoms, the hydrocarbon group being optionally substituted).

5. The method according to claim 1, wherein the cationic surfactant has an aliphatic hydrocarbon group having 12 to 30 carbon atoms, the aliphatic hydrocarbon group being optionally substituted.

6. The method according to claim 1, wherein the cationic surfactant is hexadecyltrimethylammonium chloride.

7. The method according to any one of claims 1 to 6, wherein a pore size of the filter is 0.8 μm or less.

*Fig.1*

(A)

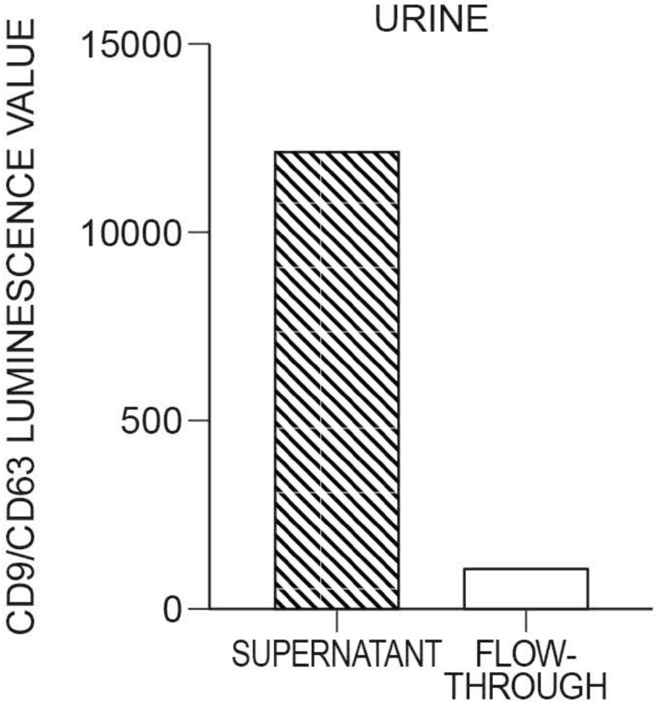
URINE
CD9/CD63 LUMINESCENCE VALUE
15000
10000
500
0
SUPERNATANT
FLOW-THROUGH

(B)

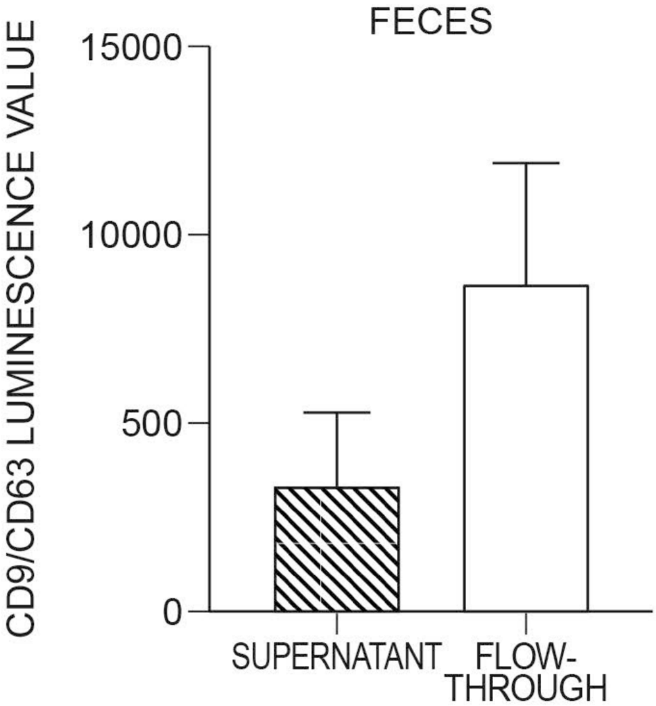
FECES
CD9/CD63 LUMINESCENCE VALUE
15000
10000
500
0
SUPERNATANT
FLOW-THROUGH

*Fig.2*

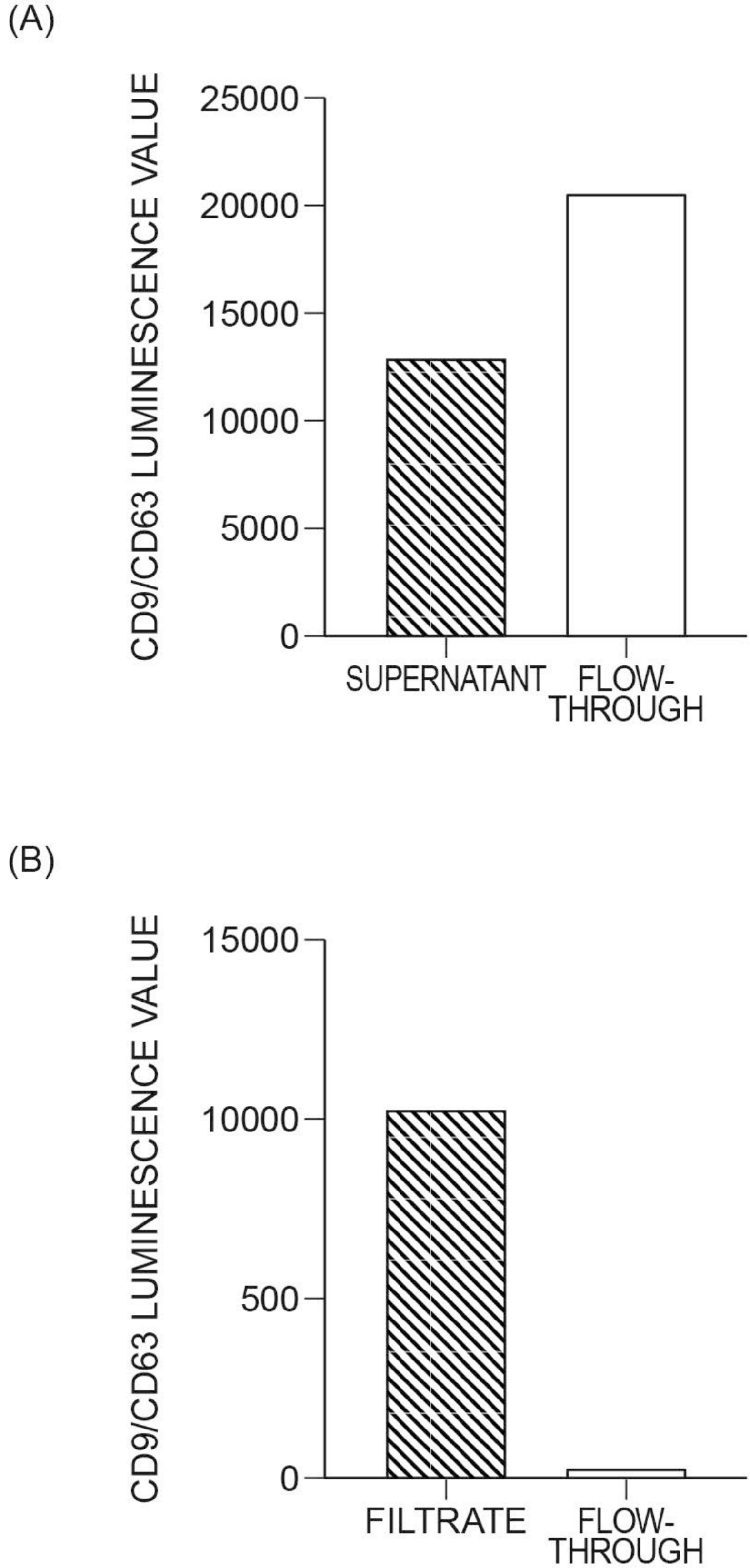
(A)
CD9/CD63 LUMINESCENCE VALUE
25000
20000
15000
10000
5000
0
SUPERNATANT
FLOW-THROUGH
(B)
CD9/CD63 LUMINESCENCE VALUE
15000
10000
500
0
FILTRATE
FLOW-THROUGH

# Fig.3

(A)

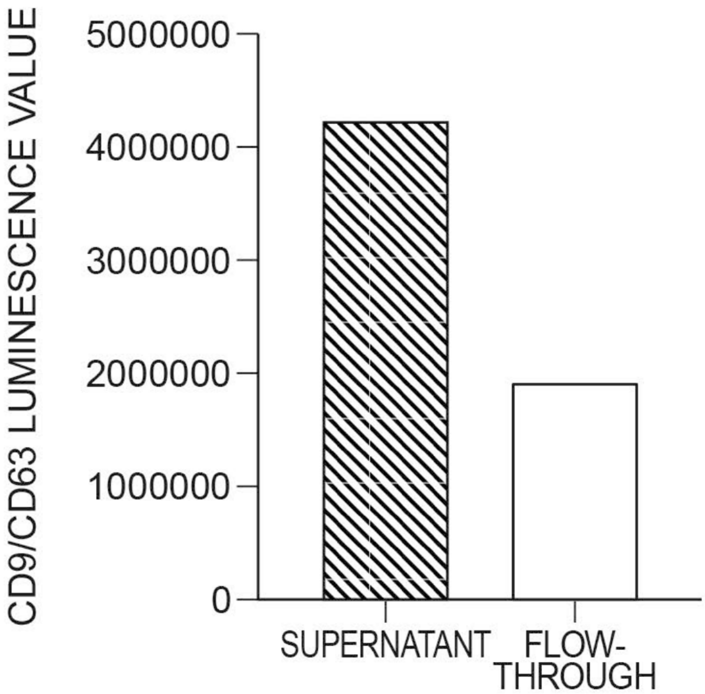
CD9/CD63 LUMINESCENCE VALUE
5000000
4000000
3000000
2000000
1000000
0
SUPERNATANT
FLOW-
THROUGH

(B)

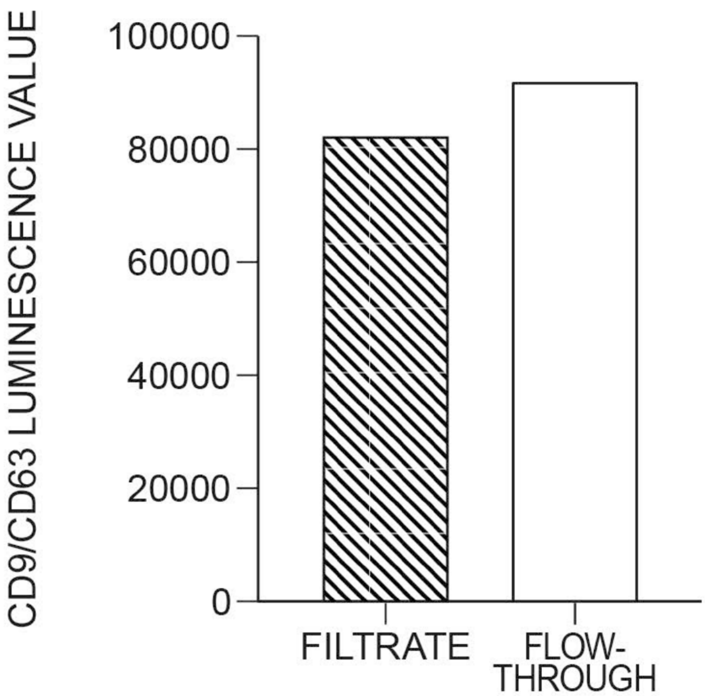
CD9/CD63 LUMINESCENCE VALUE
100000
80000
60000
40000
20000
0
FILTRATE
FLOW-
THROUGH

# Fig.4

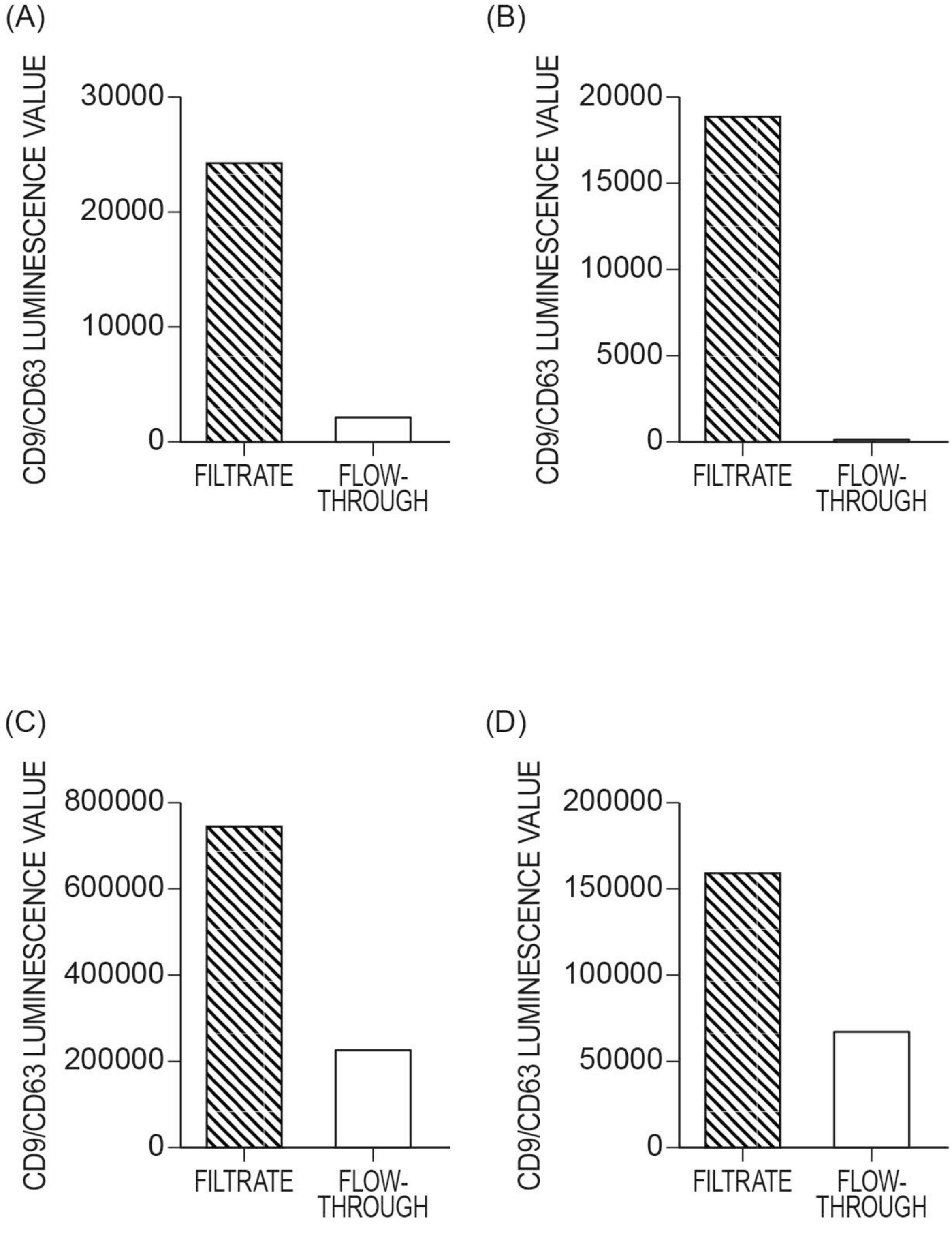
(A)
CD9/CD63 LUMINESCENCE VALUE
30000
20000
10000
0
FILTRATE
FLOW-THROUGH
(B)
CD9/CD63 LUMINESCENCE VALUE
20000
15000
10000
5000
0
FILTRATE
FLOW-THROUGH
(C)
CD9/CD63 LUMINESCENCE VALUE
800000
600000
400000
200000
0
FILTRATE
FLOW-THROUGH
(D)
CD9/CD63 LUMINESCENCE VALUE
200000
150000
100000
50000
0
FILTRATE
FLOW-THROUGH

*Fig.5*

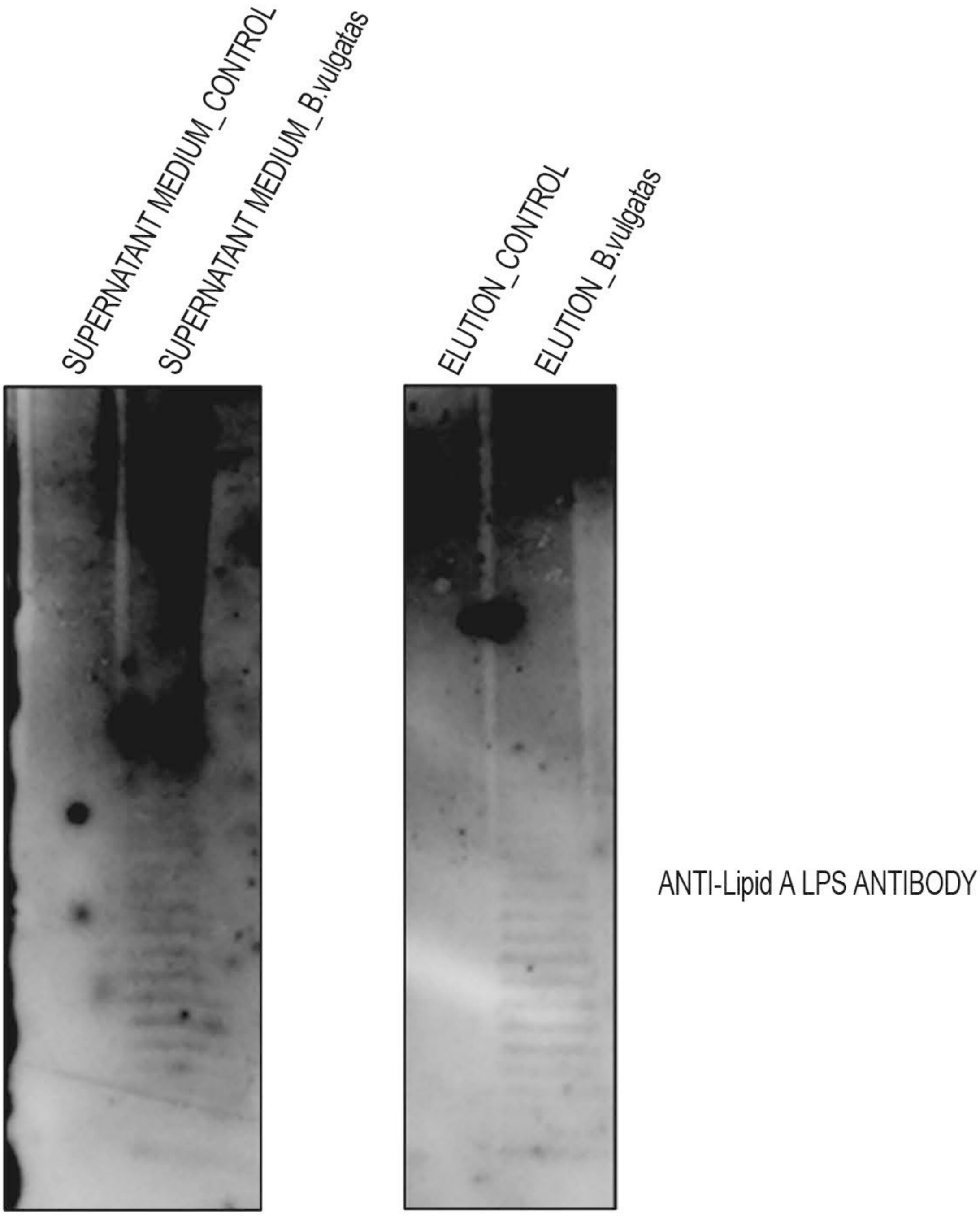
SUPERNATANT MEDIUM_CONTROL
SUPERNATANT MEDIUM_B.vulgatas
ELUTION_CONTROL
ELUTION_B.vulgatas
ANTI-Lipid A LPS ANTIBODY

Fig.6

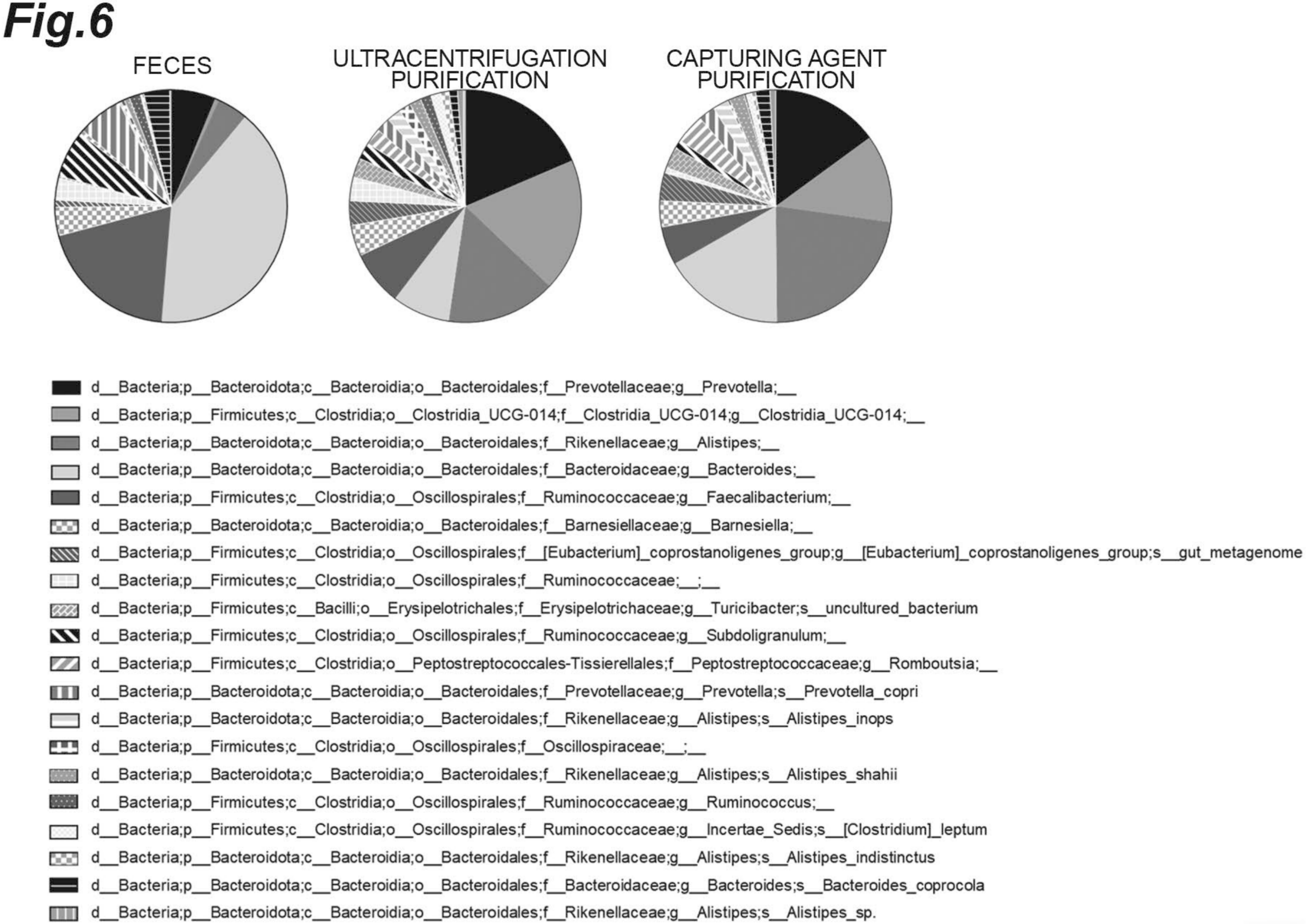
FECES
ULTRACENTRIFUGATION PURIFICATION
CAPTURING AGENT PURIFICATION
d__Bacteria;p__Bacteroidota;c__Bacteroidia;o__Bacteroidales;f__Prevotellaceae;g__Prevotella;__
d__Bacteria;p__Firmicutes;c__Clostridia;o__Clostridia_UCG-014;f__Clostridia_UCG-014;g__Clostridia_UCG-014;__
d__Bacteria;p__Bacteroidota;c__Bacteroidia;o__Bacteroidales;f__Rikenellaceae;g__Alistipes;__
d__Bacteria;p__Bacteroidota;c__Bacteroidia;o__Bacteroidales;f__Bacteroidaceae;g__Bacteroides;__
d__Bacteria;p__Firmicutes;c__Clostridia;o__Oscillospirales;f__Ruminococcaceae;g__Faecalibacterium;__
d__Bacteria;p__Bacteroidota;c__Bacteroidia;o__Bacteroidales;f__Barnesiellaceae;g__Barnesiella;__
d__Bacteria;p__Firmicutes;c__Clostridia;o__Oscillospirales;f__[Eubacterium]_coprostanoligenes_group;g__[Eubacterium]_coprostanoligenes_group;s__gut_metagenome
d__Bacteria;p__Firmicutes;c__Clostridia;o__Oscillospirales;f__Ruminococcaceae;__;__
d__Bacteria;p__Firmicutes;c__Bacilli;o__Erysipelotrichales;f__Erysipelotrichaceae;g__Turicibacter;s__uncultured_bacterium
d__Bacteria;p__Firmicutes;c__Clostridia;o__Oscillospirales;f__Ruminococcaceae;g__Subdoligranulum;__
d__Bacteria;p__Firmicutes;c__Clostridia;o__Peptostreptococcales-Tissierellales;f__Peptostreptococcaceae;g__Romboutsia;__
d__Bacteria;p__Bacteroidota;c__Bacteroidia;o__Bacteroidales;f__Prevotellaceae;g__Prevotella;s__Prevotella_copri
d__Bacteria;p__Bacteroidota;c__Bacteroidia;o__Bacteroidales;f__Rikenellaceae;g__Alistipes;s__Alistipes_inops
d__Bacteria;p__Firmicutes;c__Clostridia;o__Oscillospirales;f__Oscillospiraceae;__;__
d__Bacteria;p__Bacteroidota;c__Bacteroidia;o__Bacteroidales;f__Rikenellaceae;g__Alistipes;s__Alistipes_shahii
d__Bacteria;p__Firmicutes;c__Clostridia;o__Oscillospirales;f__Ruminococcaceae;g__Ruminococcus;__
d__Bacteria;p__Firmicutes;c__Clostridia;o__Oscillospirales;f__Ruminococcaceae;g__Incertae_Sedis;s__[Clostridium]_leptum
d__Bacteria;p__Bacteroidota;c__Bacteroidia;o__Bacteroidales;f__Rikenellaceae;g__Alistipes;s__Alistipes_indistinctus
d__Bacteria;p__Bacteroidota;c__Bacteroidia;o__Bacteroidales;f__Bacteroidaceae;g__Bacteroides;s__Bacteroides_coprocola
d__Bacteria;p__Bacteroidota;c__Bacteroidia;o__Bacteroidales;f__Rikenellaceae;g__Alistipes;s__Alistipes_sp.

## INTERNATIONAL SEARCH REPORT

International application No.
**PCT/JP2024/025934**

**A. CLASSIFICATION OF SUBJECT MATTER**

***C12N 1/02***(2006.01)i; ***C12N 5/07***(2010.01)i
FI: C12N1/02; C12N5/07

According to International Patent Classification (IPC) or to both national classification and IPC

**B. FIELDS SEARCHED**

Minimum documentation searched (classification system followed by classification symbols)

C12N1/02; C12N5/07

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | NORTHROP-ALBRECHT, E. J. et al., Assessment of extracellular vesicle isolation methods from human stool supernatant, The Journal of Extracellular Vesicles, 2022, volume11, Issue4, e12208, PP. 1-17, https://doi.org/10.1002/jev2.12208<br>abstract, 2.2, fig. 1 | 1-7 |
| A | JP 2020-513799 A (MD HEALTHCARE INC.) 21 May 2020 (2020-05-21)<br>claims 1, 2, paragraph [0035], test examples 2, 3 | 1-7 |
| A | JP 2019-205353 A (JSR CORPORATION) 05 December 2019 (2019-12-05)<br>claim 14, paragraph [0054] | 1-7 |
| A | WO 2022/211006 A1 (SANYO CHEMICAL INDUSTRIES, LTD.) 06 October 2022 (2022-10-06)<br>claims 9, 10, paragraph [0058] | 1-7 |
| A | WO 2021/251462 A1 (TOKUSHIMA UNIVERSITY) 16 December 2021 (2021-12-16)<br>claims 3, 5, 10 | 1-7 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 September 2024** | **08 October 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

INTERNATIONAL SEARCH REPORT

International application No.
**PCT/JP2024/025934**

C. DOCUMENTS CONSIDERED TO BE RELEVANT

| Category* | Citation of document, with indication, where appropriate, of the relevant passages | Relevant to claim No. |
|---|---|---|
| E, A | JP 2024-128472 A (ARTIENCE CO., LTD.) 24 September 2024 (2024-09-24) claims 1-3, 5 | 1–7 |

Form PCT/ISA/210 (second sheet) (July 2022)

**INTERNATIONAL SEARCH REPORT**
**Information on patent family members**

International application No.
**PCT/JP2024/025934**

| Patent document cited in search report | Publication date (day/month/year) | Patent family member(s) | Publication date (day/month/year) |
|---|---|---|---|
| JP 2020-513799 A | 21 May 2020 | US 2019/0390284 A1<br>claims 1, 2, paragraph [0069], test examples 2, 3<br>WO 2019/164230 A1<br>EP 3567125 A1<br>KR 10-2019-0100868 A<br>CN 110462066 A | |
| JP 2019-205353 A | 05 December 2019 | WO 2018/062263 A1 | |
| WO 2022/211006 A1 | 06 October 2022 | US 2024/0201055 A1<br>claims 9, 10, paragraph [0111]<br>EP 4317399 A1<br>CN 117295945 A | |
| WO 2021/251462 A1 | 16 December 2021 | US 2023/0221298 A1<br>claims 3, 5, 10<br>EP 4166944 A1 | |
| JP 2024-128472 A | 24 September 2024 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021251462 A
- WO 2022211006 A
- JP 2003225565 A
- JP 2005075982 A
- JP 3648553 B
- JP 2003165883 A
- JP 2005095759 A


### Non-patent literature cited in the description


- **YANG C et al.** Isolation and Characterization of Exosomes from Mouse Feces.. *Bio Protoc.*, 20 April 2020, vol. 10 (8), e3584